# EUROPEAN PATENT APPLICATION

(11) **EP 1 318 195 A1**
(43) Date of publication of application: **11.06.2003**
(21) Application number: 01204762.7
(22) Date of filing: 10.12.2001
(51) Int. Cl.: C12N 15/12, C07K 16/00, G01N 33/50, C12N 5/10, C07K 1/14, A61K 39/395, A61K 47/48, C12N 15/10

(54) **A structure for presenting desired peptide sequences**

(71) Applicant: CatchMabs B.V., 6708 PW Wageningen (NL)
(72) Inventor: Houtzager, Erwin, 3958 XD, Amerongen (NL); Vijn, Irma Maria Caecilia Vijn, 6721 ZJ, Bennekom (NL); Sijmons, Peter Christiaan, 1075 GK, Amsterdam (NL)
(74) Representative: Prins, Adrianus Willem

(57) **Abstract**

The binding properties of proteinaceous molecules to their targets was found to be manipulatable not only at the level of the sequence of the binding part on the proteinaceous molecule but also at the level of the structure of the proteinaceous molecules itself. Core sequences are identified that can be used and manipulated to obtain the desired binding properties of a binding peptide. The present invention further provides means and methods for selecting and designing such cores and binding peptides. The invention further provides uses of such proteinaceous molecules.

## Description

The invention relates to methods and means for providing binding molecules with improved properties, be it in binding or other properties, as well as the novel binding molecules themselves.

The invention further relates to methods applying these molecules in all their versatility.

In modern biotechnology, one of the most promising and in a number of cases proven applications relies on affinity of proteinaceous molecules for all kinds of substances and/or targets. Proteinaceous binding molecules have been applied in purification of substances from mixtures, in diagnostic assays for a wide array of substances, as well as in the preparation of pharmaceuticals, etc. Typically, naturally occurring proteinaceous molecules, such as immunoglobulins (or other members of the immunoglobulin superfamily) as well as receptors and enzymes have been used. Also peptides derived from such molecules have been used.

The use of existing (modified) natural molecules of course provides a limited source of properties that evolution has bestowed on these molecules. This is one of the reasons that these molecules have not been applied in all the areas where their use can be envisaged. Also, because evolution always results in a compromise between the different functions of the naturally occurring binding molecules, these molecules are not optimized for their envisaged use.

Typically, the art has moved in the direction of altering binding properties of existing (modified) binding molecules. In techniques such as phage display of (single chain) antibodies almost any binding specificity can be obtained. However, the binding regions are all presented in the same context. Thus the combination of binding region and its context is often still not optimal, limiting the use of the proteinaceous binding molecules in the art.

The present invention provides a versatile context for presenting desired affinity regions. The present invention provides a structural context that is designed based on a common structural element (called a core structure) that has been identified herein to occur in numerous binding proteins. This so called common core has now been produced as a novel proteinaceous molecule that can be provided with one or more desired affinity regions.

This proteinaceous structure does not rely on any amino acid sequence, but only on common structural elements. It can be adapted by providing different amino acid sequences and/or amino acid residues in sequences for the intended application. It can also be adapted to the needs of the particular affinity region to be displayed. The invention thus also provides libraries of both structural contexts and affinity regions to be combined to obtain an optimal proteinaceous binding molecule for a desired purpose.

Thus the invention provides a synthetic or recombinant proteinaceous molecule comprising a binding peptide and a core, said core comprising a b-barrel comprising at least 4 strands, wherein said b-barrel comprises at least two b-sheets, wherein each of said b-sheet comprises two of said strands and wherein said binding peptide is a peptide connecting two strands in said b-barrel and wherein said binding peptide is outside its natural context. We have identified this core structure in many proteins, ranging from galactosidase to human (and e.g. camel ) antibodies with all kinds of molecules in between. Nature has apparently designed this structural element for presenting desired peptide sequences. We have now produced this core in an isolated form, as well as many variants thereof that still have the same or similar structural elements. These novel structures can be used in all applications where other binding molecules are used and even beyond those applications as explained herein. The structure comprising one affinity region (desired peptide sequence) and two b-sheets forming one b-barrel is the most basic form of the invented proteinaceous binding molecules. (proteinaceous means that they are in essence amino acid sequences, but that side chains and/or groups of all kinds may be present; it is of course possible, since the amino acid sequence is of less relevance for the structure to design other molecule of non proteinaceous nature that have the same orientation is space and can present peptidic affintiy regions; the orientation in space is the important parameter). The invention also discloses optimised core structures in which less stable amino acids are replaced by more stable residues (or vice versa) according to the desired purpose. Of course other substitutions or even amino acid sequences completely unrelated to existing structures are included, since, once again, the important parameter is the orientation of the molecule in space. According to the invention it is preferred to apply a more advanced core structure than the basic structure, because both binding properties and structural properties can be designed better and with more predictive value. Thus the invention preferably provides a proteinaceous molecule according the invention wherein said b-barrel comprises at least 5 strands, wherein at least one of said sheets comprises 3 of said strands, more preferably a proteinaceous molecule according to the invention, wherein said b-barrel comprises at least 6 strands, wherein at least two of said sheets comprises 3 of said strands. Variations wherein one sheet comprises only two strands are of course also possible. In an alternative embodiment the invention provides a proteinaceous molecule according to the invention, wherein said b-barrel comprises at least 7 strands, wherein at least one of said sheets comprises 4 of said strands.

Alternatively the invention provides a proteinaceous molecule according to the invention wherein said b-barrel comprises at least 8 strands, wherein at least one of said sheets comprises 4 of said strands.

In another embodiment a proteinaceous molecule according to the invention, wherein said b-barrel comprises at least 9 strands, wherein at least one of said sheets comprises 4 of said strands is provided. In the core structure there is a more open side where nature displays affinity regions and a more closed side, where connecting sequences are present. Preferably at least on affinity region is located at said more open side.

Thus the invention provides a proteinaceous molecule according to the invention, wherein said binding peptide connects two strands of said b-barrel on the open side of said barrel. Although the location of the desired peptide sequence (affinity region) may be anywhere between two strands, it is preferred that the desired peptide sequence connects the two sheets of the barrel. Thus the invention provides a proteinaceous molecule according to the invention, wherein said binding peptide connects said at least two b-sheets of said barrel. Although one affinity region may suffice it is preferred that more affinity regions are present to arrive at a better binding molecule. Preferably, these regions are arranged such that they can cooperate in binding (e.g. both on the open side of the barrel). Thus the invention provides a proteinaceous molecule according to the invention, which comprises at least one further binding peptide. A successful core element in nature is the one having three affinity regions and three connecting regions. This core in its isolated form is a preferred embodiment of the present invention. However, because of the versatility of the presently invented binding molecules, the connecting sequences on the less open side of the barrel can be used as affinity regions as well. Also other exposed sequences can be affinity regions, for instance sequences exposed to the exterior of the core element. This way a very small bispecific binding molecule is obtained. Thus the invention provides a proteinaceous molecule according the invention, which comprises at least 4 binding peptides.

Bispecific herein means that the binding molecule has the possibility to bind to two target molecules (the same or different). As already stated it is an object of the present invention to optimise binding molecules both in the binding properties and the structural properties (such as stability under different circumstances (temperature,pH, etc), the antigenicity, etc.). This is done, according tot the invention by taking at least one nucleic acid according to the invention (encoding a proteinaceous binding molecule according to the invention) and mutating either the encoded structural regions or the affinity regions or both and testing whether a molecule with desired binding properties and structural properties has been obtained. Thus the invention provides a method for identifying a proteinaceous molecule with an altered binding property, comprising introducing an alteration in the core of proteinaceous molecules according to the invention, and selecting from said proteinaceous molecules, a proteinaceous molecule with an altered binding property, as well as a method for identifying a proteinaceous molecule with an altered structural property, comprising introducing an alteration in the core of proteinaceous molecules according to the invention, and selecting from said proteinaceous molecules, a proteinaceous molecule with an altered structural property. These alterations can vary in kind, an example being a post-translational modification. The person skilled in the art can design other relevant mutations.

As explained the mutation would typically be made by mutating the encoding nucleic acid and expressing said nucleic acid in a suitable system, which may be bacterial, eukaryotic or even cell-free. Once selected one can of course use other systems than the selection system.

The invention also provides methods for producing nucleic acids encoding proteinaceous binding molecules according to the invention, such as a method for producing a nucleic acid encoding a proteinacous molecule capable of displaying at least one desired peptide sequence comprising providing a nucleid acid sequence encoding at least a first and second structural region separated by a nucleic acid sequence encoding said desired peptide sequence or a region where such a sequence can be inserted and mutating said nucleic acid encoding said first and second structural regions to obtain a desired nucleic acid encoding said proteinacous molecule capable of displaying at least one desired peptide sequence and preferably a method for displaying a desired peptide sequence, providing a nucleic acid encoding at least a two b-sheets, said , said b-sheets forming a b-barrel, said nucleic acid comprising a region for inserting a sequence encoding said desired peptide sequence, inserting a nucleic acid sequence comprising a desired peptide sequence, and expressing said nucleid acid whereby said b sheets are obtainable by a method as described above. The invention further provides the application of the novel binding molecules in all fields where binding molecules have been envisaged until today, such as separation of substances from mixtures, typically complex biological mixtures, such as body fluids or secretion fluids, such as blood or milk, or serum or whey.

Of course pharmaceutical uses and diagnostic uses are clear to the person skilled in the art. In diagnostic uses labels may be attached to the molecules of the invention. In pharmaceutical uses other moieties can be coupled to the molecules of the invention. In both cases this may be chemically or through recombinant fusion. Diagnostic applications and pharmaceutical applications have been described in the art in great detail for conventional binding molecules. For the novel binding molecules according tot the invention no further explanation is necessary for the person skilled in the art. Gene therapy in a targeting format is one of the many examples wherein a binding molecule according to the invention is provided on the gene delivery vehicle (which may be viral (adenovirus, retrovirus, adeno associated virus or lentivirus, etc.) or non viral (liposomes and the like). Genes to be delivered are well known in the art.

The gene delivery vehicle can also encode a binding molecule according to the invention to be delivered to a target, possibly fused to a toxic moiety. Conjugates of toxic moieties to binding molecules are also well known in the art and are included for the novel binding molecules of the invention.

The invention will be explained in more detail in the following detailed description.

The present invention relates to the design, construction, production, screening and use of proteins that contain one or more regions that may be involved in molecular binding. The invention also relates to naturally occurring proteins provided with artificial binding domains, re-modelled natural occurring proteins provided with extra structural components and provided with one or more artificial binding sites, re-modelled natural occurring proteins disposed of some elements (structural or others) provided with one or more artificial binding sites, artificial proteins containing a standardized core structure motif provided with one or more binding sites. All such proteins are called VAPs (Versatile Affinity Proteins) herein. The invention further relates to novel VAPs identified according to the methods of the invention and the transfer of binding sites on naturally occurring proteins that contain a similar core structure. 3D modelling of such natural occurring proteins can be desired before transfer in order to restore or ensure antigen binding capabilities by the affinity regions present on the selected VAP. Further, the invention relates to processes that use selected VAPs, as described in the invention, for purification, removal, masking, liberation, inhibition, stimulation, capturing, etc,of the chosen ligand capable of being bound by the selected VAP(s).

### LIGAND BINDING PROTEINS

Many naturally occurring proteins that contain a (putative) molecular binding site comprise two functionally different regions: The actual displayed binding region and the region(s) that is (are) wrapped around the molecular binding site or pocket, called the Core Structure (CS) also called scaffold herein. These two regions are different in function, structure, composition and physical properties. The CS structures ensures a stable 3 dimensional conformation for the whole protein, and act as a steppingstone for the actual recognition region. Two functional different classes of ligand binding proteins can be discriminated. This discrimination is based upon the presence of a genetically variable or invariable ligand binding region. In general, the invariable ligand binding proteins contain a fixed number, a fixed composition and an invariable sequence of amino acids in the binding pocket in a cell of that species. Examples of such proteins are all cell adhesion molecules, e.g. N-CAM and V-CAM, the enzyme families, e.g. kinases and proteases and the family of growth receptors,e.g EGF-R, bFGF-R. In contrast, the genetically variable class of ligand binding proteins is under control of an active genetic shuffling-, mutational or rearrangement mechanism enabling an organism or cell to change the number, composition and sequence of amino acids in, and possibly around, the binding pocket. Exmples of these are all types of light and heavy chain of antibodies, B-cell receptor light and heavy chains and T-cell receptor alfa, beta, gamma and delta chains. The molecular constitution of wild type CS's can vary to a large extent. For example, Zinc finger containing DNA binding molecules contain a totally different CS (looking at the amino acid composition) than antibodies although both proteins are able to bind to a specific target.

### SCAFFOLDS (CORE STRUCTURES) AND LIGAND BINDING DOMAINS

### Antibodies obtained via immunizations

The class of ligand binding proteins that express variable (putative) antigen binding domains has been shown to be of great value in the search for ligand binding proteins. The classical approach to generate ligand binding proteins makes use of the animal immune system. This system is involved in the protection of an organism against foreign substances. One way of recognizing, binding and clearing the organism of such foreign highly diverse substances is the generation of antibodies against these molecules. The immune system is able to select and multiply antibody producing cells that recognize an antigen. This process can also be mimicked by means of active immunizations. After a series of immunizations antibodies may be formed that recognize and bind the antigen. The possible number of antibodies that can be formed due to genetic rearrangements and mutations, exceeds the number of 1040. However, in practice, a smaller number of antibody types will be screened and optimized by the immune system. The isolation of the correct antibody producing cells and subsequent immortalization of these cells or, alternatively, cloning of the selected antibody genes directly, antigen-antibody pairs can be conserved for future (commercial and non-commercial) use.

The use of antibodies obtained this way is restricted only to a limited number of applications. The structure of animal antibodies is different than antibodies found in human. The introduction of animal derived antibodies in humans will almost certainly cause immune responses adversing the effect of the introduced antibody (e.g. HAMA reaction). As it is not allowed to actively immunize men for commercial purposes, it is not or only rarely possible to obtain human antibodies this way. Because of these disadvantages methods have been developed to bypass the generation of animal specific antibodies. One example is the removal of the mouse immune system and the introduction of the human immune system in such mouse. All antibodies produced after immunization are of human origin. However the use of animal has also a couple of important disadvantages. First, animal care has a growing attention from ethologists, investigators, public opinion and government. Immunization belongs to a painful and stressful operation and must be prevented as much as possible. Second, immunizations do not always produce antibodies or do not always produce antibodies that contain required features such as binding strength, antigen specificity, etc. The reason for this can be multiple: the immune system missed by co-incidence such a putative antibody; the initially formed antibody appeared to be toxic or harmful; the initially formed antibody also recognizes animal specific molecules and consequently the cells that produce such antibodies will be destroyed; or the epitope cannot be mapped by the immune system (this can have several reasons).

### Otherwise obtained antibodies

It is clear, as discussed above, that immunization procedures may result in the formation of ligand binding proteins but their use is limited, inflexible and uncontrollable. The invention of methods for the bacterial production of antibody fragments (Skerra and Pluckthun, 1988; Better et al., 1988) provided new powerful tools to circumvent the use of animals and immunization procedures. It is has been shown that cloned antibody fragments, (frameworks, affinity regions and combinations of these) can be expressed in artificial systems, enabling the modulation and production of antibodies and derivatives (Fab, VL, VH, scFv and VHH) that recognize a (putative) specific target in vitro. New efficient selection technologies and improved degeneration strategies directed the development of huge artificial (among which human) antibody fragment libraries. Such libraries potentially contain antibodies fragments that can bind one or more ligands of choice. These putative ligand specific antibodies can be retrieved by screening and selection procedures. Thus, ligand binding proteins of specific targets can be engineered and retrieved without the use of animal immunizations.

### Other immunoglobulin superfamily derived scaffolds

Although most energy and effort is put in the development and optimization of natural derived or copied human antibody derived libraries, other scaffolds have also been described as succesful scaffolds as carriers for one or more ligand binding domains. Examples of scaffolds based on natural occurring antibodies encompass minibodies (Pessi et al., 1993), Camelidae VHH proteins (Davies and Riechmann, 1994; Hamers-Casterman et al., 1993) and soluble VH variants (Dimasi et al., 1997; Lauwereys et al., 1998). Two other natural occurring proteins that have been used for affinity region insertions are also member of the immunoglobulin superfamily: the T-cell receptor chains (Holler et al., 2000) and fibronectin domain-3 regions (Koide et al., 1998). The two T-cell receptor chains can hold three affinity regions each while for the fibronectin region the investigators described only two regions. Non-immunoglobulin derived scaffolds Besides immunoglobulin domain derived scaffolds, non-immunoglobulin domain containing scaffolds have been investigated. All proteins investigated contain only one protein chain and one to four affinity related regions. Smith and his colleagues (1998) reported the use of knottins (a group of small disulfide bonded proteins) as a scaffold. They successfully created a library based on knottins that had 7 mutational amino acids. Although the stability and length of the proteins are excellent, the low number of amino acids that can be randomized and the singularity of the affinity region make knottin proteins not very powerful. Ku and Schultz (1995) successfully introduced two randomized regions in the four-helix-bundle structure of cytochrome b562. However, selected binders were shown to bind with micromolar Kd values instead of the required nanomolar or even better range. Another alternate framework that has been used belongs to the tendamistat family of proteins. McConnell and Hoess (1995) demonstrated that alpha-amylase inhibitor (74 amino acid beta-sheet protein) from Streptomyces tendae could serve as a scaffold for ligand binding libraries. Two domains were shown to accept degenerated regions and function in ligand binding. The size and properties of the binders showed that tendamistats could function very well as ligand mimickers, called mimetopes. This option has now been exploited. Lipocalin proteins have also been shown to be succesful scaffolds for a maximum of four affinity regions (Beste et al., 1999; Skerra, 2000 BBA; Skerra, 2001 RMB). Lipocalins are involved in the binding of small molecules like retinoids, arachidonic acid and several different steroids. Each lipocalin has a specialized region that recognizes and binds one or more specific ligands. Skerra (2001) used the lipocalin RBP and BBP to introduce variable regions at the site of the ligand binding domain. After the construction of a library and successive screening, the investigators were able to isolate and characterize several unique binders with nanomolar specificity for the chosen ligands. It is currently not known how effective lipocalins can be produced in bacteria or fungal cells. The size of lipocalins (about 170 amino acids) is pretty large in relation to VHH chains (about 100 amino acids) which might be too large for industrial applications.

The VAPs of the invention can be used in an enormous variety of applications, from therapeutics to antibiotics, from detection reagents to purification modules, etc. etc. In each application the environment and the downstream applications determines the features that a ligand binding protein should have, e.g. temperature stability, protease resistance, tags, etc. What ever the choice of the scaffolds is, all have their own unique properties. Some properties can be advantageous for certain applications while others are unacceptable. For large scale industrial commercial uses it is crucial that scaffolds contain a modular design in order to be able to mutate, remove, insert and swap regions easily and quick. Modularity make it possible to optimize for required properties via standardized procedures and it allows domain exchange programs, e.g. exchange of pre-made cassettes. As optimal modular scaffold genes should meet certain features, they have to be designed and synthetically constructed while it is very unlikely that natural retrieved genes contains such features.

Besides modularity there are several other properties that should be present or just absent in the scaffold gene or protein. All scaffold systems that are based on frameworks that are present in natural proteins inherit also their natural properties. These properties have been optimized by evolutionary forces for the system in which this specific protein acts. Specific properties encompass for example codon usage, codon frequency, expression levels, folding patterns and cystein bridge formation. Industrial commercial production of such proteins, however, demands optimal expression, translation and folding to achieve economic profits. Not only should the genetic information be compatible and acceptable for the production organism, protein properties should also be optimal for the type of application. Such properties can be heat sensitivity, pH sensitivity, salt concentration sensitivity, proteolytic sensitivity, stability, purification possibilities, and many others.

Thus, to be of practical use in affinity processes, specific binding activity alone is not sufficient. The specific binding agent must also be capable of being linked to a solid phase such as a carrier material in a column, an insoluble bead, a plastic, metal or paper surface or any other useful surface. Ideally, this linkage is achievable without any adverse effects on the specific binding activity. Therefore the linkage is preferably accomplished with regions in the VAP molecule that are relatively remote from the specific affinity regions.

An important embodiment of the invention is an affinity-absorbant material comprising a specific binding agent immobilised on a porous silica or the like, the specific binding agent comprising a selection of VAP molecules.
A particularly important embodiment of the invention is an affinity-absorbant material comprising a special binding agent immobilised on a porous carrier material, such as silica or an inert, rigid polymer or the like, having a pore size of at least 30A but not greater than 1000A, wherein the specific binding agent comprises a selection of VAP molecules. Preferably, the carrier has a pore size of at least 60A. Preferably, the pore size is not greater than 500A, and more preferably, not greater than 300A.

The pore size of a carrier medium such as silica or inert polymers can be determined using e.g. standard size exclusion techniques or other published methods. The nominal pore size is often referred to as the mean pore diameter, and is expressed as a function of pore volume and surface area, as calculated by the Wheeler equation (MPD= (40,000 x pore volume)/surface area. The pore volume and surface area can be determined by standard nitrogen absorption methods of Brunauer, Emmett and Teller.

Products in which VAPs can be applied in a way that leaves the VAPs present up to, and also including, the end product, have examples from a very wide range of products. But also in processes where the VAPs are immobilized and preferably can be regenerated for recycled use, the major advantage of VAPs is fully exploited, i.e. the relative low cost of VAPs that makes them especially suitable for large scale applications, for which large quantities of the affinity bodies need to be used. The list below is given to indicate the scope of applications and is in no way limiting. Product or process examples with possible applications in brackets are;
(1) industrial (food) processing such as the processing of whey, tomato pomace, citrus fruits, etc. or processes related to bulk raw materials of agricultural origin such as the extraction of starch, oil and fats, proteins, fibers, sugars etc. from bulk crops such as, but not limited to; potato, corn, rice, wheat, soybean, cotton, sunflower, sugarbeet, sugarcane, tapioca, rape. Other examples of large process streams are found in the diary-related industries e.g. during cheese and butter manufacturing. As the VAPs can be used in line with existing processing steps and the VAPs do not end up in the final product as a result of their irreversible immobilisation to support-materials, they are exceptionally suited for the large scale industrial environments that are customary in agro-foodprocessing industries.
   In a more detailed example, the whey fraction that is the result of the cheese manufacturing processes contain a relatively large number of low-abundant proteins that have important biological functions, e.g. during the development of neonates, as natural antibiotics, food-additives etc. Examples of such proteins are lactoferrin, lactoperoxidase, lysozyme, angiogenine, insulin-like growth factors (IGF), insulin receptor, IGF-binding proteins, transforming growth factors (TGF), bound- and soluble TGF-receptors, epidermal growth factor (EGF), EGF-receptor ligands, interleukine-1 receptor antagonist. Another subclass of valuable compounds that can be recovered from whey are the immunoregulatory peptides that are present in milk or colostrum. Also specific VAPs can be selected for the recovery of hormones from whey. Examples of hormones that are present in milk are; prolactin, somatostatin, oxytocin, luteinizing hormone-releasing hormone, thyroid-stimulating hormone, thyroxine, calcitonin, estrogen, progesterone. Other examples where the VAPs are exceptionally suitable, are large scale affinity chromatography applications where purification of a target compound is limited for economic reasons; the cost of purification becomes too high in relation to the price of the purified compound. A very significant cost reduction can often be achieved through a reduction of the number of purification steps by a far more selective purification step that is based on specific affinities such as displayed by VAPs. In some examples, single step purifications are even feasible, where the target compound is directly purified to acceptable levels from the originating process stream (e.g. single step enzyme purification from extracellular culture media or cell extracts of industrial production microorganisms or other cell-based production methods).
(2) edible consumer products such as ice-cream, oil-based products such as oils, margarines, dressings and mayonaisse, other processed foodproducts as soups, sauces, pre-fabricated meals, soft-drinks, beer, wine, etc. (preservation and prevention of spoilage, through direct antibiotic activity or selective inhibition of enzymes, protecting sensitive motives during processing, e.g. from enzymes or compounds that influence quality of end products through its presence in an active form, or for enzymes that preferrably needs to be active down-stream of a denaturing process step and where the binding with a specific VAPs would prevent the active site of the enzyme to be denatured) controlled release of flavours and odours, molecular mimics to mask or enhance flavours and odours e.g. masking or removing bitter components in beer brewing industries, removal of pesticides or other contaminants.
(3) personal care products such as shampoos, hair-dying liquids, washing liquids, laundry detergents, gels as applied in different forms such as powders, paste, tablet or liquid form etc. (anti-microbial activity for inhibition of dandruff or other skin-related microbes, anti-microbial activity for toothpastes and mouthwashes, increased specificity for stain-removing enzymes in detergents, stabilizing labile enzymes in soaps or detergents to increase e.g. temperature or pH stability, increased binding activity for hair-dye products, inhibiting enzymes that cause body odours, either in skin applications or in clothing accessories such as shoe-inlays, hygiene tissues)
(4) non-food applications such as printing inks, glues, paints, paper, hygiene tissues etc. (surface-specific inks, glues, paints etcetera for surfaces that are otherwise difficult to print e.g. most current plastics, but especially those that are based on polyofines bottles or containers, or for surfaces where highly specific binding is required, e.g. lithographic processes in electronic chip manufacturing, authentication of value papers, etc)
(5) environmental protection processes such as water purification, bioremediation, clean-up of process waters, concentration of contaminants (removal of microorganisms, viruses, organic pollutants in water purification plants or e.g. green-house water recycling systems, removal of biological hazards from air-ventilation ducts)
(6) animal feed products in dry or wet forms (removal, masking or otherwise inhibiting the effects of anti-nutritional factors that often occur in feed components both for cattle and fish farming, notably protease inhibitors or negative factors such as phytic acid, addition of VAPs as antimicrobial agents to replace current antibiotics with protein-based antibiotics) Although the preferred embodiments of this patent include industrial processes, the use of VAPs in a manner of affinity chromatography is certainly not limited to these applications. On the "low volume / high value" side of the scale, a variety of applications is feasible for pharmaceutical, diagnostic and research purposes where price is of lesser importance for application, but the availability of VAPs against ligands that are notoriously difficult to raise antibodies against in classical immune responses. Also the small size and high stability will provide "low volume / high value" applications were VAPs are superior to conventional antibodies or fragments thereof.
(7) pharmaceutical aplications where VAPs can be used as therapeutics themselves, particularly when the core is designed to resemble a natural occurring protein, or to identify and design proper affinity regions and/or core regions for therapeutics.
(8) diagnostic applications where VAPs, as a result of their 3D structure that differ in essential ways from commonly used antibodies or antibody fragments, may detect a different class of molecules. Examples are the detection of infectious prions, where the mutation causing the infectious state is buried inside the native molecule. Coventional antibodies can only discriminate the infectious form under denatured conditions, while the small and exposed AR's of VAPs are able to recognize more inwardly placed peptide sequences.
(9) research applications where VAPs are bound to e.g. plate surfaces or tissues to increase detection levels, localize specific compounds on a fixed surface, fix tracer molecules in position etc. or where selected genes that code for specific VAPs are either transiently, continuously or in a controlled manner expressed by translating said genes in a cellular environment, and where through its targeted expression functional knock-outs of target molecules are formed. For example mimicking a receptor ligand may interfere with normal signal-transduction pathways, or VAPs that function as enzyme inhibitors may interfere with metabolic pathways or metabolic routing.

Diverse as the above examples are, there are commonalities in the ways that VAPs can be applied, as is illustrated by the following categories that form a matrix in combination with the applications:
1. affinity chromatography where VAPs are immobilized on an appropriate support e.g. in chromatography columns that can be used in line, in series or in carroussel configurations for fully continuous operation. Also pipes, tubes, in line filters etc. can be lined with immobilized VAPs. The support material on which the VAPs can be immobilized can be chosen to fit the process requirements in terms of compression stability, flow characteristics, chemical inertness, temperature-, pH- and solvent stability etc. Relatively incompressible carriers are preferred, especially silica or rigid inert polymers. These have important advantages for use in industrial-scale affinity chromatography because they can be packed in columns operable at substantially higher pressures than can be applied to softer carrier materials such as agarose. Coupling procedures for binding proteins to such diverse support materials are well-known. After charging the column with the process stream of choice, the bound ligands can be desorbed from the immobilized VAPs through well-known procedures such as changes in pH or salt concentrations after which the VAPs can be regenerated for a new cycle. The high stability of selected VAPs makes them exceptionally suitable for such repeated cycles, thus improving the cost efficiency of such recovery and purification procedures. The principles and versatility of affinity chromatography have been widely described in thousands of different applications.
2. insoluble beads are a different form of affinity chromatography where the support material on which VAPs are immobilized are not fixed in position but are available as beads from for example , silica, metal, magnetic particles, resins and the like. can be mixed in process streams to bind specific ligands in e.g. fluidised beds or stirred tanks, after which the beads can be seperated from the process stream in simple procedures using gravity, magnetism, filters etc.
3. coagulation of target ligands by crosslinking the ligands with VAPs, thereby reducing their solubility and concentrating the ligands through precipitation. For this purpose, VAPs should be bivalent, i.e. at least two AR's must be constructed on either side of the scaffold. The two AR's can have the same molecular target but wo different molecular targets are preferred to provide the cross-linking or coaggulation effects.
4. masking of specific molecules to protect sensitive motives during processing steps, to increase the stability of the target ligand for adverse pH, temperature or solvent conditions, or to increase the resistance against deteriorating or degrading enzymes. Other functional effects of molecular masking can be the masking of volatile molecules to alter the sensory perception of such molecules. In contrast the slow and conditional release of such molecules from VAPs can be invisaged in more down-stream processing steps, during consumption or digestion or after targeting the VAPs-ligand complex to appropriate sites for biomedical or research applications. Also molecular mimics of volatile compounds using VAPs with specific receptor binding capacity can be used to mask odours from consumer products.
5. coating of insoluble materials with VAPs to provide highly specific surface affinity properties or to bind VAPs or potential fusion products (i.e. products that are chemically bound to the VAPs or, in case of protein, are co-translated along with the VAPs in such manner that the specicifity of the VAPs remains unchanged) to specific surfaces. Examples are the use of VAPs to immobilize specific molecules to e.g. tissues, on plates etc. to increase detection levels, localize specific compounds on a fixed surface, fix tracer molecules in position etc.

Certainly not all natural CS's are interesting from a commercial and/or industrial point of view. For example, the stability and sensitivity of the whole protein should meet the requirements that go along with the proposed application. Ligand bindig proteins in an acidic environment are not per se useful in high salt or high temperature environments. It is not possible to design one scaffold that has all possible features to function as a one for all scaffold. For example, there are applications that require proteolytic insensitive scaffolds while other applications require specific protease cleavage sites in the scaffold. For these and many other applications it is not possible to design one scaffold that meets all requirements. However, most differences in scaffold function have to do with properties of the amino acid that are exposed on the outside of the protein. For example pH effect, proteolytic recognition and cleavage, solubility, expression levels, antigenicity and many more features mostly have to do with amino acid side chain properties instead of the core of the scaffold and the backbone. Changing the properties of these amino acids will typically have a minor effect on the overall structure of a scaffold. Thus is possible to change application forms by changing the quality of amino acids located on the outside of the scaffold or of those amino acids that are not involved in ligand binding or structure determination. With this in mind it is possible to design and construct scaffolds that can be used in multiple kinds of ligand binding environments without changing the properties and spatial position of the ligand binding domain. Consequence of this idea is that affinity domains can be swapped from one scaffold to another scaffold, if the scaffolds contain similar CS's, without losing their specificity for the ligand. We call this swapping technology Modular Affinity and Scaffold Transfer technology (MAST technology).

### Adapting scaffolds and affinity regions through mutations.

One method to introduce random mutations in pieces of DNA is the introduction of inosine residues in DNA fragments. Inosine is widely used as a residue in sequencing methods and degenerated primers. Inosine is a form of deoxy-nucleotide-tri-phosphate that can be incorporated in DNA fragments without disturbing the overall DNA structure but it lacks side groups that are responsible for H-H bridge formation. Typically, adenosine can form 2 H-H bridges with thymidine while cytosine can form 3 H-H bridges with guanidine their pairs are opposite positioned. Therefore inosine can be found opposite to A, C, G or T. The effect of inosine in DNA strands is that it will not contribute to the melting temperature of the DNA fragment. If inosine is introduced in DNA strands cellular DNA repair mechanisms will try to correct this and replace the inosine will the corresponding complementary required nucleotide of the opposite DNA strand. However under in vitro conditions, environmental systems determine the acceptance, incorporation or removal of inosine residues. For example, proof reading DNA polymerases are able to correct DNA strands for inosine residues but non-proof reading polymerases lack this property.
In DNA amplification reactions inosine residues can be introduced by at random during the formation of a new strand of DNA. Most non-proof reading polymerases (even thermostable ones) can also, by chance, incorporate inosine residues, instead of one of the four regular dNTPs, at random positions during the second strand assembly. During further steps a random regular nucleotide can be placed at the base site opposite to the inosine residue. The number of mutations is Gaussian distributed. Factors that can influence the relative number of mutations are PCR cycli number, concentration dITP, concentration regular dNTPs, polymerase properties, DNA concentration and PCR buffer composition. An optimized procedure can be applied to every stretch of nucleotides flanked by one or more primer regions.
Inosine based mutational strategies have been reported for a couple of target sequences. The inosine procedure has a couple of explicit important features: it does not introduce frameshifts, it keeps the original length of the DNA fragments, it is unbiased for the mutational position, it is (relatively) unbiased for the mutational type (dNTP type), easy to perform, controllable and it is applicable to every part of DNA that can be amplified. The effects of the mutated DNA can be: change in DNA properties (e.g. promoter elements), RNA stability and most important for our purposes, changes in amino acid composition and sequence of the DNA fragment. By influencing the number of mutations and the domains of mutations, new protein domain properties can be generated. By controlling the relative number of changes it is possible to keep the overall amino acid composition. This way structural important amino acids or stretches of amino acids within the corresponding mutated domain can be kept for a certain percentage of the obtained clones. However, the random introduction of inosine can also by coincidence result in the introduction of undesired stop codons. By controlling PCR conditions it is possible to restrict the average number of stop codons. In addition, selection procedures (frame and stop codon dependent) and the use of specific strains that can overrule specific stop codons, e.g. amber or ochre mutations in specific E.coli strains, can at least partly compensate for the effect of the putative introduced stop codons.
The consequence that only a limited number of amino acids will changed creates a new approach in the generation of randomization in for example affinity regions (e.g. CDR3). Now structural information and/or critical residues or properties at certain positions, in at least a significant percentage of clones formed using dITP mutations, can be conserved and can be extremely important in building high quality antibody libraries.
The combination of a method for producing in frame mutations within certain pre-determined regions using PCR or other techniques that include inosine residues and the possibility to (functionally) screen large numbers of mutants make an excellent and powerful combination not only in optimizing affinity regions in antibodies but also in other affinity regions or even in the optimization in the functionality or specificity of proteinous domains. In addition the combination of these techniques can be used to optimize functional domains in stretches of DNA or RNA. For example promoter, UTR, leaders, structural units, transcription initiation and transcriptional termination, translational effects, etc.
The size of the region that can be affected by the inosine mutational method is not restricted as long as the DNA fragment can be amplified.

In a specific example, it is possible now to generate structures, based on a template, that have new improved or adapted properties that were not present in the original template. Optimal temperture sensitivity, optimal protease resistance, optimal ligand binding, optimal ligand specificity, optimal pH sensitivity, optimal solubility, optimal expression, optimal translation, optimal secretion, optimal codon usage, optimal functionality, etc in which the term optimal represents the optimal configuration for a certain application (both positive and negative).

### CORE STRUCTURE DEVELOPMENT

In commercial industrial applications it is very interesting to use single chain peptides, instead of multiple chain peptides because of low costs and high efficiency of such peptides in production processes. One example that could be used in industrial applications are the VHH antibodies. Such antibodies are very stable, can have high specificities and are relatively small. However, the scaffold has evolutionarily been optimized for an immune dependent function but not for industrial applications. In addition, the highly diverse pool of framework regions that are present in one pool of antibodies prevents the use of modular optimization methods. Therefore a new scaffold was designed based on the favourable stability of VHH proteins.
3D-modelling and comparative modelling software was used to design a scaffold that meets the requirements of versatile affinity proteins (VAPs). Because it is not possible yet to predict protein structures, protein stability and other features, it is necessary to test the computer designed and modelled scaffolds. Multiple primary scaffolds were constructed and pooled. All computer designed proteins are just an estimated guess. One mutation or multiple amino acid changes in the primary scaffold may make it a successful scaffold or make it function even better than predicted. To accomplish this the constructed primary scaffolds are subjected to a mild mutation program.

Hereto the inosine dependent mutational technology is applied. Inosine insertions at random positions can ultimately change the amino acid compositions and amino acid sequences of the primary scaffolds. This way new (secondary) scaffolds are generated. In order to test the functionality, stability and other required or desired features of the scaffolds, a set of known affinity regions are inserted in the secondary scaffolds. All functional scaffolds that display the inserted affinity regions in a correct fashion will be able to bind to the ligand that corresponds to the inserted affinity regions. Phage diplay techniques help to conserve the genetic and phenotypic information of the scaffolds. After panning procedures optimal and functional scaffolds are retrieved. These scaffolds can be used for the insertion of new affinity regions.

### INITIAL AFFINITY REGIONS FOR LIBRARY CONSTRUCTION

In the present invention new and unique affinity regions are required. Affinity regions can be obtained from natural sources, degenerated primers or stacked DNA triplets. All of these sources have certain important limitations as described above. In our new setting we designed a new and strongly improved source of affinity regions which have less restrictions, can be used in modular systems, are extremely flexible in use and optimization, are fast and easy to generate and modulate, have a low percentage of stop codons, have an extremely low percentage of frameshifts and wherein important structural features will be conserved in a large fraction of the new formed clones and new structural elements can be introduced.

The major important affinity region (CDR3) in both light and heavy chain in normal antibodies has a average length between 11 (mouse) and 13 (human) amino acids. Because in such antibodies the CDR3 in light and heavy chain cooperatively function as antigen binder, the strength of such a binding is a result of both regions together. In contrast, the binding of antigens by VHH antibodies (Camelidae) is a result of one CDR3 regions due to the absence of a light chain. With an estimated average length of 16 amino acids these CDR3 regions are significantly longer than regular CDR3 regions (Mol. Immunol. Bang Vu et al., 1997, 34, 1121-1131). It can be emphasized that long or multiple CDR3 region have potentially more interaction sites with the ligand and can therefore be more specific and bind with more strength. Average lengths of the major affinity region(s) should preferably be about 16 amino acids. In order to cover as much as possible potentially functional CDR lengths the major affinity region can vary between 1 and 50 or even more amino acids. As the structure and the structural classes of CDR3 regions (like for CDR1 and CDR2) have not been clarified and understood it is not possible to design long affinity regions in a way that the position and properties of crucial amino acids are correct. Therefore, most libraries were supplied with completely degenerated region in order to find at least some correct regions. In the invention we describe the use of natural occurring VHH CDR3 region as a template for new affinity regions. CDR3 regions were isolated from mRNA coding for VHH antibodies originating to various animals of the camelidae group by means of PCR techniques. Next this pool of about 108 different CDR3 regions, which differ in the coding for amino acid composition, amino acid sequence, putative structural classes and length, is subjected to a mutational process by PCR amplification that includes dITP in the reaction mixture. The result is that the very most products will differ from the original templates and thus contain coding regions that potentially have different affinity region. Other very important consequences are that the products keep their length, the pool keeps their length distribution, a significant part will keep structural important information while others might form non-natural classes of structures, the products do not or only rarely contain frame shifts and a the majority of the products will lack stop codons. These new affinity regions can be cloned into the selected scaffolds. The newly constructed library can be subjected to screening procedures similarly as regular libraries known by an experienced user in the field of the art.

### AFFINITY MATURATION

After one or more selection rounds, an enriched population of VAPs is formed that recognizesthe ligand selected for. In order to obtain better, different or otherwise changed VAPs against the ligand(s), the VAP coding regions or parts thereof can be the subject of a mutational program by several means. Beside chain shuffling, error prone PCR stratagies or mutator strains, the inosine dependent mutational technology can be applied. Due to the modular nature of the VAPs, parts of the VAP can be used in the last mentioned mutational technology. Several stratagies are possible: First, the whole VAP or VAPs can be used as a template. Second, only one or more affinity region can be mutated. Third framework regions can be mutated. Fourth, fragments throughout the VAP can be used as a template. Of course itterative processes can be applied to change more regions. The average number of mutations can be varied by changing PCR conditions. This way every desired region can be mutated and every desired level of mutation numbers can be applied independently. After the mutational procedure, the new formed pool of VAPs can be re-screened and re-selected in order to find new and improved VAPs against the ligand(s). The process of maturation can be re-started and re-applied as much rounds as necessary.
The effect of inosine dependent mutational is that not only affinity regions 1 and 2 with desired affinities and specificities can be found but also that minor changes in the selected affinity region 3 can be inttroduced. It has been shown that mutational programs in this major ligand binding region can strongly increase ligand binding properties. In conclusion, the invention described here is extremely powerful in the maturation phase.

### EXAMPLES

### Example 1.

### INTRODUCING NEW BINDING AND/OR OTHER PROPERTIES OF OBTAINED CAMEL ANTIBODIES.

Lama pacos lymphocytes are obtained by isolating lymphocytes via a discontinue gradient of Ficol (Pharmacia Biotech). Five ml heparin lama blood is diluted with 5 ml phosphate buffered saline (PBS) and applied on the top of 4 ml Ficol. After centrifugation for 20 minutes at 1000g without brakes an interphase will form mainly containing lyphocytes. This fraction is isolated and diluted with PBS to 50 ml and the cell are pelleted at 1000g by centrifugation for 10 minutes. The supernatant is discarded and the cells are lysed in Tri-Pure (Roche Diagnostics) using a teflon piston and the mRNA is isolated according to the manufactures protocol. Two microgram mRNA is reverse transcribed using oligo-dT primers and AMV reverse transcriptase (Roche-Diagnostics) according to the manufactures procedure. Five microliter of the obtained cDNA is directly used in a PCR amplification reaction for 30 cycles at 93°C 15 seconds, 50°C 20 seconds, 70°C 60 seconds with a mixture containing 250nM forward and backward primers (ordered at Genset), 100micromolar PCR grade dNTPs (Roche-Diagnostics), 2.5 units High Fidelity Taq mix (Roche-Diagnostics) and the supplied buffer including magnesium sulphate. The produced products are separated on a 1% agarose Tris Buffered EDTA (TBE) gel and cut out and purified using Qiagen PCR purification methods. The isolated PCR products are used in mutational PCR reactions. The cycles are repeated for 10 times using the same temperature and time conditions as described above in the RT-PCR reaction. The mixtures contain dITP concentrations varying from 1 micromolar to 200 micromolar, 10 micromolar dNTPs, 2.5 unit Taq (Roche-Diagnostics), 5' biotin labelled primers at 250nM each (same primers sequences as mentioned above). The formed PCR products are purified using a biotin purification kit according to the manufactures procedures. Inosines are replaced by adding short random phosphorylated oligomers to the template. Mixture is heated and cooled down to room temperture. After the addition of buffer, dNTPs, DNA T4 ligase and the klenow fragment DNA polymerase and an incubation at room temperature a proof reading polymerase is added to replace the inosines of the template DNA that is now paired with a non-inosine containing duplicate build from the random oligos and filled in with the polymerase. Isolated products are re-PCRed for 10 cycles according to the first PCR schema with the exception that the primers now contain an unique restriction site at the 5 prime end to enable unidirectional cloning. After amplification the biotin labelled template used in this PCR reaction is removed using the above mentioned method. Amplified products are purified using Qiagen PCR purification kit and digested with appropriate restriction enzymes. Next the gel separated, cut out and repurified DNA fragments are ligated in a bla-containing (ampicillin resistance gene) phage display vector expression vector with the M13 g3 protein in frame along with a His tag, a detection tag (HA or VSV) and a proteolytic site (trypsin) which are located in between the PCR cloning site and the g3 open reading frame. After electroporation of the ligation products in TG1 E.coli, cells are grown on plates that contain 4% glucose, ampicillin and 2*TY-agar. Harvested cells are used to infect with the kanamycine bearing VCSM13 helper phage (Stratagene). Helper phage infected cells are grown in 2*TY/ kanamycine/ ampicillin on a 200rpm shaking platform at 30°C for 8 hours. Next the bacteria are removed by pelleting at 20000g at 4°C for 30 minutes. The supernatant is filtered through a 0.45 micrometer PVDF filter membrane (MilliPore). Poly-ethylene-glycol and NaCl are added to a final concentration of the flow through to respectively 4% and 0.5M. This way phages will precipitate on ice and can be obtained by centrifugation. The phage pellet is solved in 50% glycerol/50% PBS and stored at -20°C. Panning is performed by diluting about 10¹³ stored phages in 10ml PBS/0.5% gelatine. The antigen is coated on 2 types of immunotubes, low charged and high charged tubes (Greiner). After a blocking period of 2-8 hours at 4°C the phage mixture is added and incubated on a slowly rotating platform at 4°C for 60 minutes. The immunotubes are washed 5 times with PBS/0.5% gelatine and 5 times with PBS/0.05% tween-20. Bound phages are eluted with 0.1M glycine pH 2.2 (HCl) for 1 minute and the eluted fluid is neutralized with Tris 8.8. Ten volumes of XL1-blue cells at OD 0.1-0.3 are infected with the neutralized phage solution. After infection the cells are plated on 2*TY agar/ ampicillin/tetracyclin/4%glucose plates and grown overnight at 37°C. Re-panning can be performed until a desired number of cycles or colonies or individual or pooled colonies can be screened for inserts and/or binding capacity and antigen specificity. Growth of individual or pooled clones and the formation of chimeric phages is basically the same as growing a whole bank of phages. After phage precipitation the phage are added to coated and blocked ELISA plates in order to bind to the target antigen followed by extensive washing steps similarly as described above. Anti-M13-HRP antibodies (Pharmacia Biotech) are added (1:5000) to test the number of bound phages (in relation to control wells in the ELISA plate). After a 30 minute binding period and a tenfold wash procedure with PBS/0.05% tween-20, the HRP-coated antibodies against M13 phages are detected using TMB or other ELISA-reader and HRP compatible reagents. The binding properties are read in means of staining and staining intensity. Strong signals indicate strong binding. Selected clones can be re-mutated using the above described dITP method or other mutational strategies in order to improve binding and/or other properties.

### Example 2.

### DESIGN OF A MAXIMAL PRIMARY SCAFFOLD

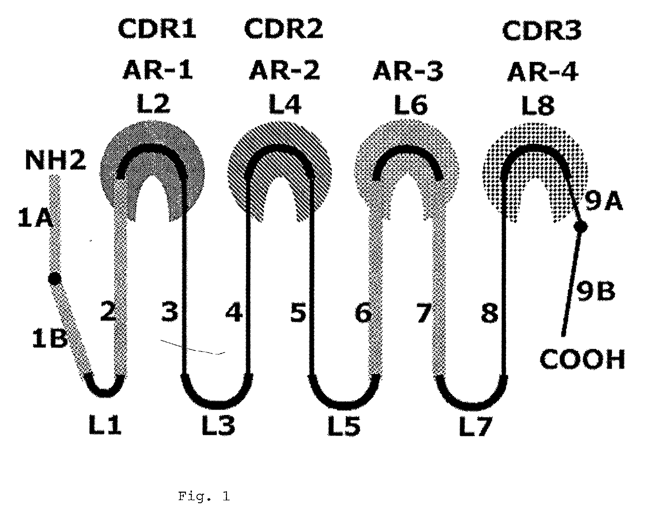

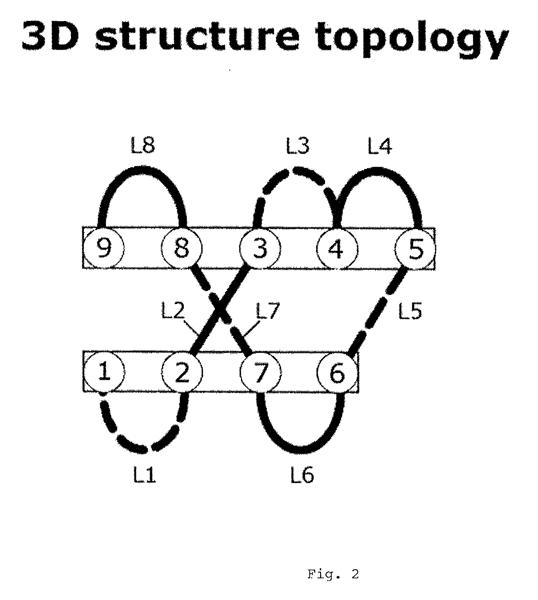

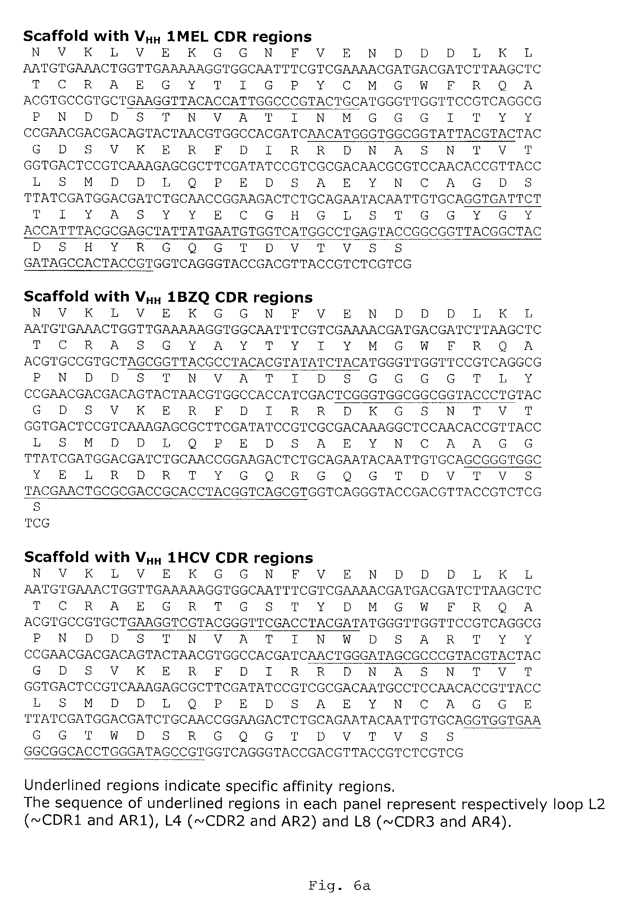

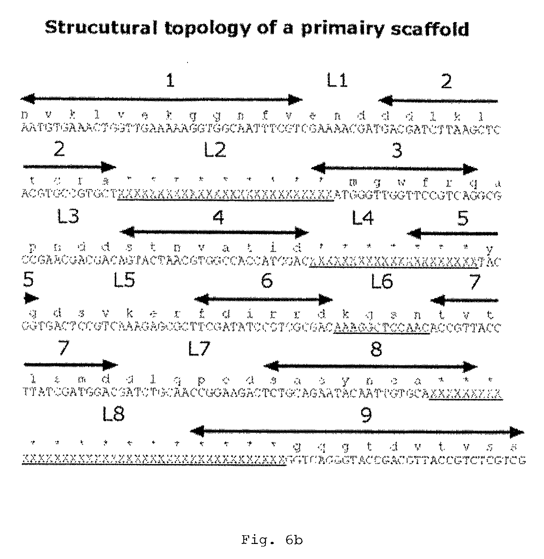

A primary scaffold is designed as a template to generate secondary scaffolds with improved properties or features by mutational strategies. A primary scaffold design has to meet some desired criteria: first it should be highly soluble, very stable and easy to fold. The solubility of proteins can be changed for example by changing amino acids that have side chains that are exposed to the environment. Hydrophilic amino acids like lysine, aspartic acid, arginine, histidine, serine, threonine or glutamic acid can improve the solubility. The stability and folding properties can be improved by the addition of intramolecular bonds. The most powerful bond is found in sulphide-bridges between two opposed cysteine residues. However, bacterial cells, in contrast to prokaryotic cells like yeast cells, can have problems with cysteine bridge formations and therefore only a limited number of cysteine bridges should be incorporated in proteins that have to be expressed in bacterial cells. Because the used scaffold is structurally based upon immunoglobular domains and because at least some bacterial proteins contain such structures or deviations of these, one can incorporate the structural and amino acid sequence knowledge of these proteins into new scaffolds that are based upon these structures. Using Modeller 6.0, Insight II individual or stretches of amino acid changes were checked for stability prognoses in comparative modelling situations. Especially the outer amino acids of the structures were initially modelled. Vector NTI suite 7.0 software was used to design the corresponding DNA sequences including codon optimizations for E.coli cells and the incorporation of desired restriction sites. Variable regions from human mouse and lama (especially VHH) have been used as a template to design a new scaffold. This scaffold therefore contains 9 beta-elements (see figure 1 and 2) arranged in 2 beta-sheets. The four even numbered loops can form a binding region on one side of the scaffold (for example as found in antibodies) while the odd numbered loops on the other side of the scaffold can be used for a binding region for another or even the same molecule. From all models that were created using comparative modelling software the next primary scaffold with 9 beta-elements was chosen based on energy minimization, potential folding properties, potential solubility and potential stability: N V K L V E K G G N F V E N D D D L K L 
AATGTGAAACTGGTTGAAAAAGGTGGCAATTTCGTCGAAAACGATGACGATCTTAAGCTC
T C R A E G * * * * * Y C M G W F R Q A
ACGTGCCGTGCTGAAGGTNNNNNNNNNNNNNNNTACTGCATGGGTTGGTTCCGTCAGGCG
P N D D S T N V A T I L * G S T Y Y G D
CCGAACGACGACAGTACTAACGTGGCCACGATCTTANNNGGGAGCACGTACTACGGTGAC
S V K E R F D I R R D * * * N T V T L S
TCCGTCAAAGAGCGCTTCGATATCCGTCGCGACNNNNNNNNNAACACCGTTACCTTATCG
M D D L Q P E D S A E Y N C A G S * Y
ATGGACGATCTGCAACCGGAAGACTCTGCAGAATACAATTGTGCAGGTTCT(NNN)XTAC
H Y R G Q G T D V T V S S
CACTACCGTGGTCAGGGTACCGACGTTACCGTCTCGTCG
 The underlined regions indicate the four affinity regions as depicted in figure 1. Although variations in length can occur in all affinity regions, the fourth underlined affinity region can tolerate extensive length variations (1-more than 40 amino acids). In figure 6 an example of this primary scaffold is depicted which has been adapted for CDR regions with known antigen binding properties (affinity region 1,2 and 4). These affinity regions originate from lama derived VHH antibody fragment clones 1HCV, which can bind human chorionic gonadotropin (HCG), those of 1MEL, which can bind lysozym, and the CDR regions of 1BZQ, which have been shown to bind bovine RNase A. The depicted DNA sequences in figure 6b have been ordered at Genset's. The synthetically produced DNA sequence are tested for their binding properties. Therefore the DNA fragments are cloned into a phage display vector as described in example 1. Phage production and panning experiments are also performed as described above with the exception that the coating of immunotubes is done with respectively bovine RNase A, HCG or lysozym. Binding phages are eluted and individual clones are tested for their sequence. In order to improve the scaffold, a mutational program is started as described in example 1, preferably using the dITP method.

### Example 3.

### DESIGN OF A MINIMAL PRIMARY SCAFFOLD

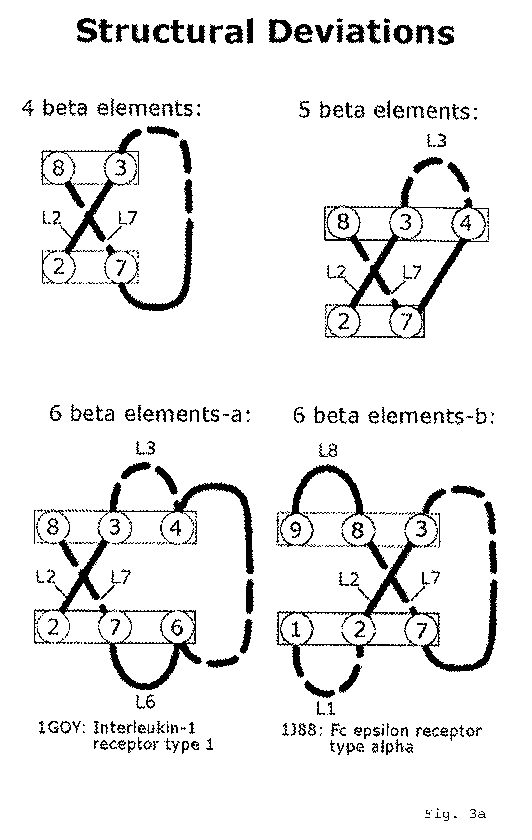

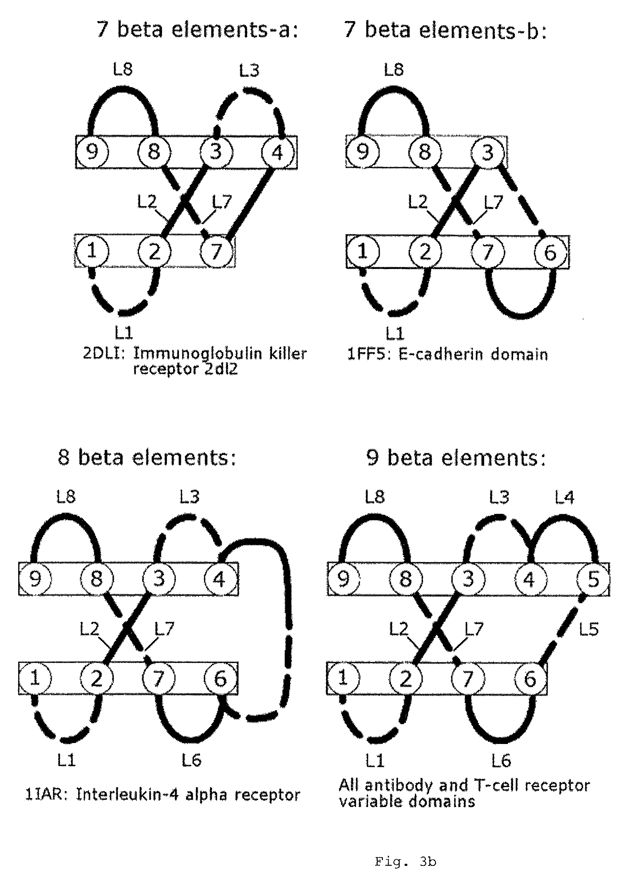

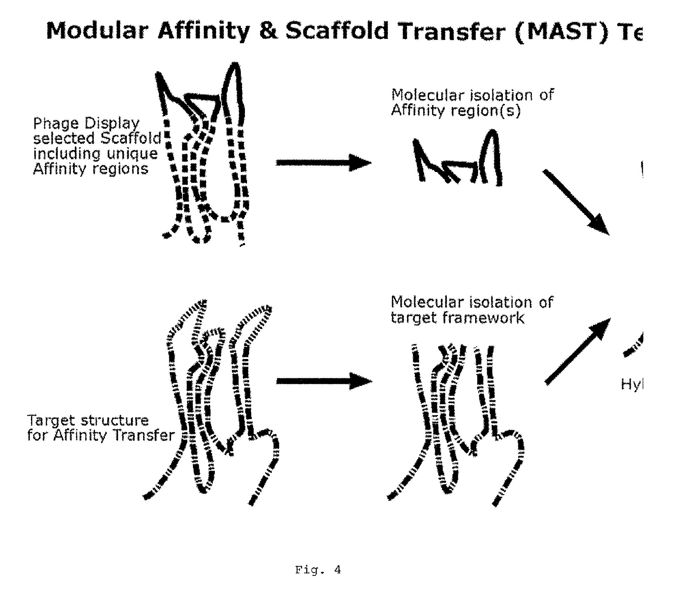

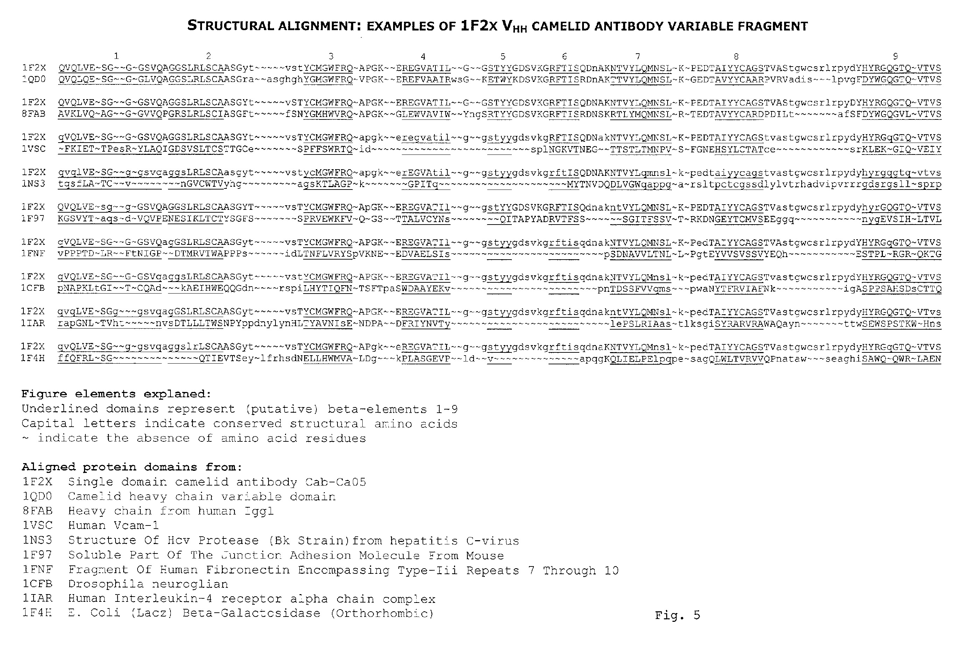

A minimal scaffold is designed according to the requirements and features as described in example 2. However now only four and five beta-elements are used in the scaffold (see figure 3). In the case of 4 beta-elements amino acids side chains of beta-elements 2, 3, 7 and 8 that are forming the mantle of the new scaffold need to be adjusted for a watery environment. The same is true for the side chains of amino acid in beta-elements 2, 4, 7 and 8 in the case of a 5 beta-elements containing scaffold. The Fc-epsilon receptor type alpha (structural code 1J88 at VAST) is used as a template for comparative modelling to design a new small scaffold consisting of 4 beta-elements. For the five beta-elements containing scaffold immunoglobulin killer receptor 2dl2 (VAST code 2DLI) is used as a structural template for modelling.

### Example 4.

### STRUCTURAL ANALYSIS OF SCAFFOLDS

In order to verify the crystal structure of scaffolds and isolated binding proteins generated via the above mentioned examples, crystallographic methods are applied. However, it is known that not all proteins can be crystallized (about 50%) and thus not all proteins can be analyzed this way. Because the core of the proteins should consist of beta-elements and beta-sheets, circular dichroism (CD) spectroscopy is used to test the presence of beta-structures.

### Example 5.

### IMPROVING PROPERTIES OF A SCAFFOLD FOR APPLICATIONS

For certain applications the properties of a scaffold need to be optimized. For example heat stability, acid tolerance or proteolytic stability can be advantageous or even required in certain environments in order to function well. A mutational and re-selection program can be applied to create a new scaffold with similar binding properties but with improved properties. In this example a selected binding protein is improved to resist proteolytic degradation in a proteolytic environment. A mixture or a cascade of proteases or the environment of the application is used to select for stability. First the selected scaffold is mutated using the above mentioned dITP methods (example 1) or other mutational methods. Next a phage display library is build from the mutated PCR products so that the new scaffolds are expressed on the outside of phages. Next the phages are added to a the desired proteolytic active environment for a certain time at the future application temperature. The remaining phages are isolated and subjected to a panning procedure like described above (example 1). After extensive washing bound phages are eluted, infected in E.coli cells that bear F-pili and grown overnight on a agar plate containing the appropriate antibiotics. Individual clones are re-checked for their new properties and sequenced. The process of mutation introduction and selection can be repeated several times or other selection conditions can be applied in further optimization rounds.

### Example 6: Random mutagenesis of multiple cloning sites (MCS) by PCR using dITP.

The MCS of vector pBluescript KS+ (Stratagene) is amplified with the T7 (5' AATACGACTCACTATAG 3') and the T3 (5' ATTAACCCTCACTAAAG 3') primer (Isogen?)in the following polymerase chain reaction (PCR): 50 ng of vector DNA is amplified by Taq DNA polymerase (0.3 units per reaction, Promega)) in a 50 ml reaction containing 50 ng of T3 and T7 primer, 2 mM MgCl2, 10mM Tris-HCl (pH 8.3), 50 mM KCl, 200 µM dNTPs (Roche Diagnostics) and with or without 200 µM dITP (Boerhinger). After 30 cycles (45 sec 94 °C, 45 sec 44 °C and 45 sec 72 °C) (Primus 96^{plus}, MWG-Biotech) a part of the PCR products are digested with the endonucleases XbaI, PstI, EcoRI, Xho I (Gibco BRL; 20 units per reaction) and run on a 2.5 % agarose gel, while the rest of the PCR products are cloned into the vector pGEMTeasy (Promega) and sequenced. The PCR products produced in the absence of dITP are digested completely, while the PCR products produced in the presence of dITP are only partly digested, showing mutagenisation of the recognition site of the endonuclease..

### Example 7: Controlled random mutagenesis of multiple cloning sites (MCS) with a different number of PCR cycli in the presence of dITP.

Amplification of the MCS of vector pBluescript KS+ is performed as in example 6 with the following modifications: the number of PCR cycles is varied from 5, 10, 20 to 30 cycles. The PCR products are treated as in example 6. The PCR products produced after 5 PCR cycles in the presence of dITP are nearly completely digested, but the PCR products produced after 30 cycles are clearly partly digested. The ratio between undigested and digested products increases with the number of PCR cycles. The different PCR products show an increase in the number of random mutations in the PCR products generated with a higher number of cycles.

### Example 8: Controlled random mutagenesis of multiple cloning sites (MCS) with a different ratio of dNTPs and dITP.

Amplification of the MCS of vector pBluescript KS+ (stratagene) is performed as in example 6 with the following modifications: The concentration of dNTPs is varied from 20 µM, 40 µM, 100 µM to 200 µM and the dITP concentration is set at 200 µM. After 30 PCR cycles the products are digested as in example 6 and separated on a 2.5 % agarose gel. The higher the ratio dITP:dNTPs the more undigested product is present. The products show more mutations when generated with a higher ratio dITP:dNTPs.

### Example 9: Controlled random mutagenesis of multiple cloning sites (MCS) with a different concentration of dITP.

Amplification of the MCS of vector pBluescript KS+ (stratagene) is performed as in example 6 with the following modifications: The concentration of dITP is varied from 0 µM, 100 µM to 200 µM and the dNTP concentration is set at either 100 µM. After 30 PCR cycles the products are digested as in example 6 and separated on a 2.5 % agarose gel. Undigested products are detected in the PCR products produced in the presence of dITP. The ratio between undigested versus digested products increases with a higher dITP concentration. The products show a higher number of mutations when produced in the presence of a higher concentration of dITP.

### Example 10: Controlled random mutagenesis of multiple cloning sites (MCS) with dITP by using different DNA polymerases.

Amplification of the MCS of vector pBluescript KS+ (stratagene) is performed as in example 6 with the following modifications: in addition to the Taq DNA polymerase DNA polymerases with proofreading such as, pwo and Deep Vent are used for amplification. The PCR products are treated as in example 6. More undigested product is detected in the products generated with DNA polymerases having no proofreading activity. The PCR products show more mutations when generated by DNA polymerases with no proofreading activity.

### Example 11: Controlled random mutagenesis of multiple cloning sites (MCS) with a different concentration of one of the dNTPs.

Amplification of the MCS of vector pBluescript KS+ (stratagene) is performed as in example 6 with the following modifications: four parallel PCR reactions are performed in which in each reaction the concentration of one of the dNTPs is lowered to 20 µM, while the concentration of the other three nucleotides is set 200 µM. The concentration of dITP is in all reactions 200 µM. The results show that preferably compatible nucleotides to the nucleotide present at a low concentration are mutated.

### Example 12: Cloning of inosine containing PCR products

Products that contain substantial numbers of inosine residues appeared hard to clone. All tested DNA polymerases fail to extend if inosine residues are present in the template. Therefore inosine residues should be replaced or strands containing inosine residues should be copied otherwise. To overcome this problem we melted inosine containing templates in the presence of phosphorylated random oligo-mers (7-20 mers). Now at primers can anneal randomly over the inosine containing spot and thus a random nucleotide can be present opposite to the inosine residue. The addition of a non-proofreading polymerase, sufficient dNTP's, ATP and a DNA ligase can result in the formation of a template based DNA copy without inosines in it. After treatment with a proof reading DNA polymerase inosines are replaced with normal nucleotides. After amplification and cloning of the products high levels of mutations can be obtained. This whole process of inosine based mutations can be repeated with or without prior cloning steps until desired levels of mutations are obtained.

### Example 13: Random mutagenesis of CDR regions

Forward and reverse primers that anneal at -20 and +20 bp, respectively, around the CDR3 region of lama derived 1HCV, 1MEL and 1BZQ are used for amplification of this CDR region in the presence of dITP as described in example 6 and 12. These PCR products are cloned and sequenced as described in example 6. Random mutations are detected in the CDR3 region.

### Example 14: Random mutagenesis of a mixture of CDR regions

Forward and reverse primers that anneal around CDR3 regions, such are used for amplification of these CDR regions in the presence of dITP as described in example 6 and 12. The PCR products are cloned and tested for binding properties as described in example 1,6 and 12. Random mutations are obviously present in at least part of all newly generated CDR3 regions.

### Example 15: Random mutagenesis of scaffold

Primers annealing at the 5 prime and 3 prime end of the scaffold sequence are used for in the presence of dITP as described in example 6 and 12. These PCR products are cloned and sequenced as described in example 6 and 12. Random mutations are detected in the amplified and cloned fragments.

### Description of the figures.

### Figure 1:

### Domain notification of immunoglobular structures.

The diagram represents a topology of a protein consisting of 9 beta-elements (indicated 1-9 from N-terminal to C-terminal). Beta elements 1,2, 6 and 7 (blocked line type) and elements 3,4,5,8 and 9 (straight line type) form two beta-sheets.

Eight loops (L1-L8) are responsible for the connection of all beta-elements. Loop 2, 4, 6 and 8 are located at the top site of the diagram and this represents the physical location of these loops in example proteins. The function of loops 2,4 and 8 in light and heavy chain variable domains is to bind antigens (marked with patterned regions). The position of L6 (also marked with a patterned region) also allows antigen binding activity, but it has not been reported yet as a region with such properties. Loops 1, 3,5 and 7 are located at the opposite site of the proteins.

### Figure 2:

### Schematic 3D topology of immunoglobular domains.

Nine beta-elements are depicted in the diagram. Each number corresponds to their position in the protein from N- to C-terminal (see figure 1). Both sets of elements 1, 2, 7 and 6 and 9, 8, 3, 4 and 5 form beta-sheets. The two beta-sheets are located in parallel to each other and are drawn from a top view perspective. The loops that connect the beta-elements are also depicted. Bold lines are connecting loops between beta-elements that are in top position while dashed lines indicate connecting loops that are located in bottom position.

### Figure 3:

### Schematic 3D-topology of scaffold domains.

Eight example topologies of protein structures that can be used for the presentation of antigen binding sites. The actual core of most scaffolds is formed by 4 beta-elements (2, 3, 7 and 8) arranged in two small beta-sheets formed by respectively elements 2 and 7 and by elements 8 and 3. The loops that connect the beta-elements are also depicted. Bold lines are connecting loops between beta-elements that are in top position while dashed lines indicate connecting loops that are located in bottom position. A connection that starts dashed and ends solid indicates a connection between a bottom and top part of beta-elements. The numbers of the beta-elements depicted in the diagram correspond to the numbers and positions mentioned in figures 1 and 2. Conserved loops are also indicated (e.g. L2 and L7) and also correspond to the loop numbers in the other figures. Some 3D-structure topologies of putative scaffolds are also present in the natural repertoire. In such cases one example is given at the bottom of the mentioned figure.

### Figure 4:

### Modular Affinity & Scaffold Transfer (MAST) Technique.

Putative antigen binding proteins that contain a core structure as described here can be used for transfer operations. Also scaffold or core structures can be used for transfer actions. The transfer operation can occur between structural identical or comparable scaffolds or cores that differ in amino acid composition. Putative affinity regions can be transferred from one scaffold or core to another scaffold or core by for example PCR, restriction digestions, DNA synthesis or other molecular techniques. The results of such transfers is depicted here in a very schematic diagram. The putative (coding) binding regions from molecule A (top part, Affinity regions) and the scaffold (coding) region of molecule B (bottom part, framework regions) can be isolated by molecular means. After recombination of both elements (hybrid structure) a new molecule appears that has binding properties of molecule A and scaffold properties of scaffold B.

### Figure 5:

### Structural alignments of naturally occurring proteins that contain at least a part of an immunoglobular structure.

Using the VAST protocol (http://www.ncbi.nlm.nih.gov/Structure/VAST/vast.shtml) it is possible to find identical or partly identical structures. VAST uses an algorithm that can predicts the presence of identical elements in pre-submitted structures with an example template structure. Here the structure of 1F2X, a functional camelid variable domain (VHH) containing an immunoglobular domain, was chosen as a template to identify natural occurring proteins in all kinds of organisms that share structural elements. Next the amino acid sequence of some of the retrieved proteins was aligned. The location of all 9 beta-elements organized in two beta-sheets is indicated by underlining. Some matched proteins contain all 9 beta-elements while others lack one or more of these. Despite the lack of one or more structural components, such proteins still form a basic common structure. Amino acid sequence comparisons show that there is hardly any conservation although the 3D-structures of these proteins is, at least partially, identical. Structural identical proteins from all kinds of organisms were retrieved this way, varying from bacteria to flies to human.

### Figure 6:

### Topology of a primary scaffold.

a) Amino acid sequence of a primary scaffold that is used to obtain optimal (secondary) scaffolds. Amino acid sequences were obtained using modeling software (Modeller 6.0 and Insight II) in combination with DNA and proteins analysis software (Vector NTI suite 7.0, InforMax). Light and heavy chain variable regions were used as a template to design this primary scaffold. The numbers (1-9) indicate beta-elements and L1-L8 indicate loops similarly as described in earlier figures. Underlined regions (L2, L4, L6 and L8) indicate affinity regions located at one site of the proteins. L2, L4 and L8 correspond to the location of CDR regions.
b) Amino acid and corresponding DNA sequences of a primary scaffold in which example CDR regions are inserted. These sets of CDR regions, depicted underlined in the figure, originate from 3 different camelid derived VHH variable regions, known as 1BZQ, 1HCV and 1MEL.

### References

Beste G, Schmidt FS, Stibora T, Skerra A. Proc Natl Acad Sci U S A. 1999 Mar Better M, Chang CP, Robinson RR, Horwitz AH. Science. 1988 May 20;240(4855):1041-3.

Davies J, Riechmann L. FEBS Lett. 1994 Feb 21;339(3):285-90.

Dimasi N, Martin F, Volpari C, Brunetti M, Biasiol G, Altamura S, Cortese R, De Francesco R, Steinkuhler C, Sollazzo M. J Virol. 1997 Oct;71(10):7461-9.

Hamers-Casterman C, Atarhouch T, Muyldermans S, Robinson G, Hamers C, Songa EB, Bendahman N, Hamers R. Nature. 1993 Jun 3;363(6428):446-8.

Lauwereys M, Arbabi Ghahroudi M, Desmyter A, Kinne J, Holzer W, De Genst E, Wyns L,Muyldermans S. EMBO J. 1998 Jul 1;17(13):3512-20.

McConnell SJ, Hoess RH. J Mol Biol. 1995 Jul 21;250(4):460-70.

Smith GP, Patel SU, Windass JD, Thornton JM, Winter G, Griffiths AD. J Mol Biol. 1998 Mar 27;277(2):317-32.

Ku J, Schultz PG. Proc Natl Acad Sci U S A. 1995 Jul 3;92(14):6552-6. 2;96(5):1898-903.

Koide A, Bailey CW, Huang X, Koide S, J. Mol. Biol. 1998 284 (4): 1141-1151

Shusta EV, Pessi A, Bianchi E, Crameri A, Venturini S, Tramontano A, Sollazzo M. Nature. 1993 Mar 25;362(6418):367-9.

Holler PD, Kieke MC, Kranz DM, Wittrup KD, Nat. Biotechnol. 2000 18(7): 754-759

Skerra A. Biochim Biophys Acta. 2000 Oct 18;1482(1-2):337-50. Review.

Skerra A. J Biotechnol. 2001 Jun;74(4):257-75.

Skerra A, Pluckthun A. Science 1988 May 20;240(4855):1038-41

## Claims

1. A synthetic or recombinant proteinaceous molecule comprising a binding peptide and a core, said core comprising a b-barrel comprising at least 4 strands, wherein said b-barrel comprises at least two b-sheets, wherein each of said b-sheet comprises two of said strands and wherein said binding peptide is a peptide connecting two strands in said b-barrel and wherein said binding peptide is outside its natural context.

2. A proteinaceous molecule according to claim 1, wherein said b-barrel comprises at least 5 strands, wherein at least one of said sheets comprises 3 of said strands.

3. A proteinaceous molecule according to claim 1 or claim 2, wherein said b-barrel comprises at least 6 strands, wherein at least two of said sheets comprises 3 of said strands.

4. A proteinaceous molecule according to any one of claims 1-3,
wherein said b-barrel comprises at least 7 strands, wherein at least one of said sheets comprises 4 of said strands.

5. A proteinaceous molecule according to any one of claims 1-4,
wherein said b-barrel comprises at least 8 strands, wherein at least one of said sheets comprises 4 of said strands.

6. A proteinaceous molecule according to any one of claims 1-5,
wherein said b-barrel comprises at least 9 strands, wherein at least one of said sheets comprises 4 of said strands.

7. A proteinaceous molecule according to any one of claims 1-6,
wherein said binding peptide connects two strands of said b-barrel on the open side of said barrel.

8. A proteinaceous molecule according to any one of claims 1-7,
wherein said binding peptide connects said at least two b-sheets of said barrel.

9. A proteinaceous molecule according to any one of claims 1-8, which comprises at least one further binding peptide.

10. A proteinaceous molecule according to any one of claims 1-9, which comprises three binding peptides and three connecting peptide sequences.

11. A proteinaceous molecule according to any one of claims 1-9, which comprises at least 4 binding peptides.

12. A proteinaceous molecule according to claim 11, wherein at least one binding peptide recognizes another target molecule than at least one of the other binding peptides.

13. A method for identifying a proteinaceous molecule with an altered binding property, comprising introducing an alteration in the core of proteinaceous molecules according to any one of claims 1-12, and selecting from said proteinaceous molecules, a proteinaceous molecule with an altered binding property.

14. A method for identifying a proteinaceous molecule with an altered structural property, comprising introducing an alteration in the core of proteinaceous molecules according to any one of claims 1-2, and selecting from said proteinaceous molecules, a proteinaceous molecule with an altered binding property.

15. A method according to claim 13 or 14, wherein said alteration comprises a post-translational modification.

16. A method according to any one of claims 13-15, wherein said alteration is introduced into a nucleic acid coding for said at least one proteinaceous molecule, the method further comprising expressing said nucleic acid in an expression system that is capable of producing said proteinaceous molecule.

17. A proteinaceous molecule obtainable by a method according to any one of claims 13-16.

18. A proteinaceous molecule according to any one of claims 1-12 or 17, which is derived from the immunoglobulin superfamily.

19. A proteinaceous molecule according to claim 18, wherein the exterior of the proteinaceous molecule is immunologically similar to the immunoglobulin superfamily molecule it was derived from.

20. A cell comprising a proteinaceous molecule according to any one of claims 1-12 or 17-19.

21. A method for producing a nucleic acid encoding a proteinacous molecule capable of displaying at least one desired peptide sequence comprising providing a nucleid acid sequence encoding at least a first and second structural region separated by a nucleic acid sequence encoding said desired peptide sequence or a region where such a sequence can be inserted and mutating said nucleic acid encoding said first and second structural regions to obtain a desired nucleic acid encoding said proteinacous molecule capable of displaying at least one desired peptide sequence.

22. A method for displaying a desired peptide sequence, providing a nucleic acid encoding at least a two b-sheets, said , said b-sheets forming a b-barrel, said nucleic acid comprising a region for inserting a sequence encoding said desired peptide sequence, inserting a nucleic acid sequence comprising a desired peptide sequence, and expressing said nucleid acid whereby said b sheets are obtainable by a method according to claim 21.

23. Use of a proteinaceous molecule according to any one of claims 1-12 or 17-19, for separating a substance from a mixture.

24. A use according to claim 23, wherein said mixture is a biological fluid.

25. A use according to claim 24, wherein said biological fluis is an excretion product of an organism.

26. A use according to claim 25, wherein said excretion product is milk or a derivative of milk.

27. A use according to claim 24, wherein said mixture is blood or a derivative thereof.

28. A proteinaceous molecule according to any one of claims 1-12 or 17-19, for use as a pharmaceutical.

29. Use of a proteinaceous molecule according to any one of claims 1-12 or 17-19, in the preparation of a pharmaceutical formulation for the treatment of a pathological condition involving unwanted proteins or cells or microorganisms.

30. Use of a proteinaceous molecule according to any one of claims 1-12 or 17-19, in the preparation of a diagnostic assay.

31. A gene delivery vehicle comprising a proteinaceous molecule according to any one of claims 1-12 or 17-19. and a gene of interest.

32. A gene delivery vehicle comprising a nucleic acid encoding proteinaceous molecule according to any one of claims 1-12 or 17-19. and a nucleic acid sequence encoding a gene of interest.

33. A proteinaceous molecule according to any one of claims 1-12 or 17-19 conjugated to a moiety of interest.

34. A proteinaceous molecule according to claim 33, wherein said moiety of interest is a toxic moiety.

35. A chromatography column comprising a proteinaceous molecule according to any one of claims 1-12 or 17-19 and a packing material.

36. A nucleic acid obtainable by the method of claim 21.

37. A nucleic acid library comprising a collection of different nucleic acids according to claim 36.

38. A nucleic acid library according to claim 37, further comprising a collection of nucleic acids encoding different affinity regions.

39. A library according to claim 37 or 38, which is an expression library.


SEQUENCE LISTING

<110> CatchMabs B.V.

<120> A structure for presenting desired peptide sequences

EPO - DG 1

<130> P58644EP00

<140> 01204762.7
<141> 2001-12-10

15. 04. 2002

<160> 22

(41)

<170> PatentIn Ver. 2.1

<210> 1
<211> 336
<212> DNA
<213> Artificial Sequence

<220>
<223> Description of Combined DNA/RNA Molecule: primary
      scaffold

<220>
<223> Description of Artificial Sequence: primary
      scaffold

<220>
<221> CDS
<222> (1)..(336)
<223> /note="Wherein N stands for unknown"

<400> 1
aat gtg aaa ctg gtt gaa aaa ggt ggc aat ttc gtc gaa aac gat gac    48
Asn Val Lys Leu Val Glu Lys Gly Gly Asn Phe Val Glu Asn Asp Asp
  1               5                  10                  15

gat ctt aag ctc acg tgc cgt gct gaa ggt nnn nnn nnn nnn nnn tac    96
Asp Leu Lys Leu Thr Cys Arg Ala Glu Gly Xaa Xaa Xaa Xaa Xaa Tyr
             20                  25                  30

tgc atg ggt tgg ttc cgt cag gcg ccg aac gac gac agt act aac gtg    144
Cys Met Gly Trp Phe Arg Gln Ala Pro Asn Asp Asp Ser Thr Asn Val
         35                  40                  45

gcc acg atc tta nnn ggg agc acg tac tac ggt gac tcc gtc aaa gag    192
Ala Thr Ile Leu Xaa Gly Ser Thr Tyr Tyr Gly Asp Ser Val Lys Glu
     50                  55                  60

cgc ttc gat atc cgt cgc gac nnn nnn nnn aac acc gtt acc tta tcg    240
Arg Phe Asp Ile Arg Arg Asp Xaa Xaa Xaa Asn Thr Val Thr Leu Ser
 65                  70                  75                  80

atg gac gat ctg caa ccg gaa gac tct gca gaa tac aat tgt gca ggt    288
Met Asp Asp Leu Gln Pro Glu Asp Ser Ala Glu Tyr Asn Cys Ala Gly
                 85                  90                  95

tct nnn tac cac tac cgt ggt cag ggt acc gac gtt acc gtc tcg tcg    336
Ser Xaa Tyr His Tyr Arg Gly Gln Gly Thr Asp Val Thr Val Ser Ser
            100                 105                 110

<210> 2
<211> 112
<212> PRT
<213> Artificial Sequence
<223> Description of Artificial Sequence: primary

scaffold

<400> 2
Asn Val Lys Leu Val Glu Lys Gly Gly Asn Phe Val Glu Asn Asp Asp
1               5                   10                  15

Asp Leu Lys Leu Thr Cys Arg Ala Glu Gly Xaa Xaa Xaa Xaa Xaa Tyr
            20                  25                  30

Cys Met Gly Trp Phe Arg Gln Ala Pro Asn Asp Asp Ser Thr Asn Val
        35                  40                  45

Ala Thr Ile Leu Xaa Gly Ser Thr Tyr Tyr Gly Asp Ser Val Lys Glu
    50                  55                  60

Arg Phe Asp Ile Arg Arg Asp Xaa Xaa Xaa Asn Thr Val Thr Leu Ser
65                  70                  75                  80

Met Asp Asp Leu Gln Pro Glu Asp Ser Ala Glu Tyr Asn Cys Ala Gly
                85                  90                  95

Ser Xaa Tyr His Tyr Arg Gly Gln Gly Thr Asp Val Thr Val Ser Ser
            100                 105                 110

<210> 3
<211> 17
<212> DNA
<213> Artificial Sequence

<220>
<223> Description of Artificial Sequence: primer T7

<220>
<221> misc_feature
<222> (1)..(17)

<400> 3
aatacgactc actatag                                                17

<210> 4
<211> 17
<212> DNA
<213> Artificial Sequence

<220>
<223> Description of Artificial Sequence: primer T3

<220>
<221> misc_feature
<222> (1)..(17)

<400> 4
attaaccctc actaaag                                                17

<210> 5
<211> 125
<212> PRT
<213> Lama

<220>
<221> SITE
<222> (1)..(125)
<223> /note="Antibody Cab-Ca05"

<400> 5
Gln Val Gln Leu Val Glu Ser Gly Gly Gly Ser Val Gln Ala Gly Gly
1               5                   10                  15

Ser Leu Arg Leu Ser Cys Ala Ala Ser Gly Tyr Thr Val Ser Thr Tyr
            20                  25                  30

Cys Met Gly Trp Phe Arg Gln Ala Pro Gly Lys Glu Arg Glu Gly Val
        35                  40                  45

Ala Thr Ile Leu Gly Gly Ser Thr Tyr Tyr Gly Asp Ser Val Lys Gly
    50                  55                  60

Arg Phe Thr Ile Ser Gln Asp Asn Ala Lys Asn Thr Val Tyr Leu Gln
65                  70                  75                  80

Met Asn Ser Leu Lys Pro Glu Asp Thr Ala Ile Tyr Tyr Cys Ala Gly
                85                  90                  95

Ser Thr Val Ala Ser Thr Gly Trp Cys Ser Arg Leu Arg Pro Tyr Asp
            100                 105                 110

Tyr His Tyr Arg Gly Gln Gly Thr Gln Val Thr Val Ser
        115                 120                 125


<210> 6
<211> 127
<212> PRT
<213> Lama

<220>
<221> SITE
<222> (1)..(127)
<223> /note="Heavy chain variable domain"

<400> 6
Gln Val Gln Leu Gln Glu Ser Gly Gly Gly Leu Val Gln Ala Gly Gly
1               5                   10                  15

Ser Leu Arg Leu Ser Cys Ala Ala Ser Gly Arg Ala Ala Ser Gly His
            20                  25                  30

Gly His Tyr Gly Met Gly Trp Phe Arg Gln Val Pro Gly Lys Glu Arg
        35                  40                  45

Glu Phe Val Ala Ala Ile Arg Trp Ser Gly Lys Glu Thr Trp Tyr Lys
    50                  55                  60

Asp Ser Val Lys Gly Arg Phe Thr Ile Ser Arg Asp Asn Ala Lys Thr
65                  70                  75                  80

Thr Val Tyr Leu Gln Met Asn Ser Leu Lys Gly Glu Asp Thr Ala Val
                85                  90                  95

Tyr Tyr Cys Ala Ala Arg Pro Val Arg Val Ala Asp Ile Ser Leu Pro
            100                 105                 110

Val Gly Phe Asp Tyr Trp Gly Gln Gly Thr Gln Val Thr Val Ser
        115                 120                 125


<210> 7
<211> 120
<212> PRT
<213> Homo sapiens

<220>

<221> SITE
<222> (1)..(120)
<223> /note="Igg1 heavy chain"

<400> 7
Ala Val Lys Leu Val Gln Ala Gly Gly Gly Val Val Gln Pro Gly Arg
1               5                   10                  15

Ser Leu Arg Leu Ser Cys Ile Ala Ser Gly Phe Thr Phe Ser Asn Tyr
            20                  25                  30

Gly Met His Trp Val Arg Gln Ala Pro Gly Lys Gly Leu Glu Trp Val
        35                  40                  45

Ala Val Ile Trp Tyr Asn Gly Ser Arg Thr Tyr Tyr Gly Asp Ser Val
    50                  55                  60

Lys Gly Arg Phe Thr Ile Ser Arg Asp Asn Ser Lys Arg Thr Leu Tyr
65                  70                  75                  80

Met Gln Met Asn Ser Leu Arg Thr Glu Asp Thr Ala Val Tyr Tyr Cys
                85                  90                  95

Ala Arg Asp Pro Asp Ile Leu Thr Ala Phe Ser Phe Asp Tyr Trp Gly
            100                 105                 110

Gln Gly Val Leu Val Thr Val Ser
        115                 120


<210> 8
<211> 89
<212> PRT
<213> Homo sapiens

<220>
<221> SITE
<222> (1)..(89)
<223> /note="Vcam-1"

<400> 8
Phe Lys Ile Glu Thr Thr Pro Glu Ser Arg Tyr Leu Ala Gln Ile Gly
1               5                   10                  15

Asp Ser Val Ser Leu Thr Cys Ser Thr Thr Gly Cys Glu Ser Pro Phe
            20                  25                  30

Phe Ser Trp Arg Thr Gln Ile Asp Ser Pro Leu Asn Gly Lys Val Thr
        35                  40                  45

Asn Glu Gly Thr Thr Ser Thr Leu Thr Met Asn Pro Val Ser Phe Gly
    50                  55                  60

Asn Glu His Ser Tyr Leu Cys Thr Ala Thr Cys Glu Ser Arg Lys Leu
65                  70                  75                  80

Glu Lys Gly Ile Gln Val Glu Ile Tyr
                85


<210> 9
<211> 92
<212> PRT
<213> Hepatitis C virus

<220>
<221> SITE
<222> (1)..(92)

<223> /note="Hcv Protease"

<400> 9
Thr Gln Ser Phe Leu Ala Thr Cys Val Asn Gly Val Cys Trp Thr Val
1               5                   10                  15

Tyr His Gly Ala Gly Ser Lys Thr Leu Ala Gly Pro Lys Gly Pro Ile
            20                  25                  30

Thr Gln Met Tyr Thr Asn Val Asp Gln Asp Leu Val Gly Trp Gln Ala
        35                  40                  45

Pro Pro Gly Ala Arg Ser Leu Thr Pro Cys Thr Cys Gly Ser Ser Asp
    50                  55                  60

Leu Tyr Leu Val Thr Arg His Ala Asp Val Ile Pro Val Arg Arg Arg
65                  70                  75                  80

Gly Asp Ser Arg Gly Ser Leu Leu Ser Pro Arg Pro
                85                  90


<210> 10
<211> 102
<212> PRT
<213> Mus musculus

<220>
<221> SITE
<222> (1)..(102)
<223> /note="Soluble part of the Junction Adhesion
      Molecule"

<400> 10
Lys Gly Ser Val Tyr Thr Ala Gln Ser Asp Val Gln Val Pro Glu Asn
1               5                   10                  15

Glu Ser Ile Lys Leu Thr Cys Thr Tyr Ser Gly Phe Ser Ser Pro Arg
            20                  25                  30

Val Glu Trp Lys Phe Val Gln Gly Ser Thr Thr Ala Leu Val Cys Tyr
        35                  40                  45

Asn Ser Gln Ile Thr Ala Pro Tyr Ala Asp Arg Val Thr Phe Ser Ser
    50                  55                  60

Ser Gly Ile Thr Phe Ser Ser Val Thr Arg Lys Asp Asn Gly Glu Tyr
65                  70                  75                  80

Thr Cys Met Val Ser Glu Glu Gly Gly Gln Asn Tyr Gly Glu Val Ser
                85                  90                  95

Ile His Leu Thr Val Leu
            100


<210> 11
<211> 91
<212> PRT
<213> Homo sapiens

<220>
<221> SITE
<222> (1)..(91)
<223> /note="Fragment of Fibronectin Encompassing
      Type-Iii repeats 7 through 10"

<400> 11

Val Pro Pro Pro Thr Asp Leu Arg Phe Thr Asn Ile Gly Pro Asp Thr
1               5                   10                  15

Met Arg Val Thr Trp Ala Pro Pro Pro Ser Ile Asp Leu Thr Asn Phe
            20                  25                  30

Leu Val Arg Tyr Ser Pro Val Lys Asn Glu Glu Asp Val Ala Glu Leu
        35                  40                  45

Ser Ile Ser Pro Ser Asp Asn Ala Val Val Leu Thr Asn Leu Leu Pro
    50                  55                  60

Gly Thr Glu Tyr Val Val Ser Val Ser Ser Val Tyr Glu Gln His Glu
65                  70                  75                  80

Ser Thr Pro Leu Arg Gly Arg Gln Lys Thr Gly
                85                  90


<210> 12
<211> 95
<212> PRT
<213> Drosophila

<220>
<221> SITE
<222> (1)..(95)
<223> /note="Neuroglian"

<400> 12
Pro Asn Ala Pro Lys Leu Thr Gly Ile Thr Cys Gln Ala Asp Lys Ala
1               5                   10                  15

Glu Ile His Trp Glu Gln Gln Gly Asp Asn Arg Ser Pro Ile Leu His
            20                  25                  30

Tyr Thr Ile Gln Phe Asn Thr Ser Phe Thr Pro Ala Ser Trp Asp Ala
        35                  40                  45

Ala Tyr Glu Lys Val Pro Asn Thr Asp Ser Ser Phe Val Val Gln Met
    50                  55                  60

Ser Pro Trp Ala Asn Tyr Thr Phe Arg Val Ile Ala Phe Asn Lys Ile
65                  70                  75                  80

Gly Ala Ser Pro Pro Ser Ala His Ser Asp Ser Cys Thr Thr Gln
                85                  90                  95


<210> 13
<211> 100
<212> PRT
<213> Homo sapiens

<220>
<221> SITE
<222> (1)..(100)
<223> /note="Interleukin-4 receptor alpha chain complex"

<400> 13
Arg Ala Pro Gly Asn Leu Thr Val His Thr Asn Val Ser Asp Thr Leu
1               5                   10                  15

Leu Leu Thr Trp Ser Asn Pro Tyr Pro Pro Asp Asn Tyr Leu Tyr Asn
            20                  25                  30

His Leu Thr Tyr Ala Val Asn Ile Ser Glu Asn Asp Pro Ala Asp Phe
        35                  40                  45

Arg Ile Tyr Asn Val Thr Tyr Leu Glu Pro Ser Leu Arg Ile Ala Ala
    50                  55                  60

Ser Thr Leu Lys Ser Gly Ile Ser Tyr Arg Ala Arg Val Arg Ala Trp
65                  70                  75                  80

Ala Gln Ala Tyr Asn Thr Thr Trp Ser Glu Trp Ser Pro Ser Thr Lys
                85                  90                  95

Trp His Asn Ser
            100


<210> 14
<211> 100
<212> PRT
<213> Escherichia coli

<220>
<221> SITE
<222> (1)..(100)
<223> /note="(Lacz) Beta-Galactosidase"

<400> 14
Phe Phe Gln Phe Arg Leu Ser Gly Gln Thr Ile Glu Val Thr Ser Glu
1               5                   10                  15

Tyr Leu Phe Arg His Ser Asp Asn Glu Leu Leu His Trp Met Val Ala
            20                  25                  30

Leu Asp Gly Lys Pro Leu Ala Ser Gly Glu Val Pro Leu Asp Val Ala
        35                  40                  45

Pro Gln Gly Lys Gln Leu Ile Glu Leu Pro Glu Leu Pro Gln Pro Glu
    50                  55                  60

Ser Ala Gly Gln Leu Trp Leu Thr Val Arg Val Val Gln Pro Asn Ala
65                  70                  75                  80

Thr Ala Trp Ser Glu Ala Gly His Ile Ser Ala Trp Gln Gln Trp Arg
                85                  90                  95

Leu Ala Glu Asn
            100


<210> 15
<211> 405
<212> DNA
<213> Artificial Sequence

<220>
<223> Description of Combined DNA/RNA Molecule: scaffold
      with Vhh 1MEL CDR regions

<220>
<223> Description of Artificial Sequence: scaffold with
      Vhh 1MEL CDR regions

<220>
<221> CDS
<222> (1)..(405)

<400> 15
aat gtg aaa ctg gtt gaa aaa ggt ggc aat ttc gtc gaa aac gat gac      48
Asn Val Lys Leu Val Glu Lys Gly Gly Asn Phe Val Glu Asn Asp Asp
1               5                   10                  15

gat ctt aag ctc acg tgc cgt gct gaa ggt tac acc att ggc ccg tac     96
Asp Leu Lys Leu Thr Cys Arg Ala Glu Gly Tyr Thr Ile Gly Pro Tyr
            20                  25                  30

tgc atg ggt tgg ttc cgt cag gcg ccg aac gac gac agt act aac gtg     144
Cys Met Gly Trp Phe Arg Gln Ala Pro Asn Asp Asp Ser Thr Asn Val
        35                  40                  45

gcc acg atc aac atg ggt ggc ggt att acg tac tac ggt gac tcc gtc     192
Ala Thr Ile Asn Met Gly Gly Gly Ile Thr Tyr Tyr Gly Asp Ser Val
    50                  55                  60

aaa gag cgc ttc gat atc cgt cgc gac aac gcg tcc aac acc gtt acc     240
Lys Glu Arg Phe Asp Ile Arg Arg Asp Asn Ala Ser Asn Thr Val Thr
65                  70                  75                  80

tta tcg atg gac gat ctg caa ccg gaa gac tct gca gaa tac aat tgt     288
Leu Ser Met Asp Asp Leu Gln Pro Glu Asp Ser Ala Glu Tyr Asn Cys
                85                  90                  95

gca ggt gat tct acc att tac gcg agc tat tat gaa tgt ggt cat ggc     336
Ala Gly Asp Ser Thr Ile Tyr Ala Ser Tyr Tyr Glu Cys Gly His Gly
            100                 105                 110

ctg agt acc ggc ggt tac ggc tac gat agc cac tac cgt ggt cag ggt     384
Leu Ser Thr Gly Gly Tyr Gly Tyr Asp Ser His Tyr Arg Gly Gln Gly
        115                 120                 125

acc gac gtt acc gtc tcg tcg                                         405
Thr Asp Val Thr Val Ser Ser
    130                 135


<210> 16
<211> 135
<212> PRT
<213> Artificial Sequence
<223> Description of Artificial Sequence: scaffold with
      Vhh 1MEL CDR regions

<400> 16
Asn Val Lys Leu Val Glu Lys Gly Gly Asn Phe Val Glu Asn Asp Asp
1               5                   10                  15

Asp Leu Lys Leu Thr Cys Arg Ala Glu Gly Tyr Thr Ile Gly Pro Tyr
            20                  25                  30

Cys Met Gly Trp Phe Arg Gln Ala Pro Asn Asp Asp Ser Thr Asn Val
        35                  40                  45

Ala Thr Ile Asn Met Gly Gly Gly Ile Thr Tyr Tyr Gly Asp Ser Val
    50                  55                  60

Lys Glu Arg Phe Asp Ile Arg Arg Asp Asn Ala Ser Asn Thr Val Thr
65                  70                  75                  80

Leu Ser Met Asp Asp Leu Gln Pro Glu Asp Ser Ala Glu Tyr Asn Cys
                85                  90                  95

Ala Gly Asp Ser Thr Ile Tyr Ala Ser Tyr Tyr Glu Cys Gly His Gly
            100                 105                 110

Leu Ser Thr Gly Gly Tyr Gly Tyr Asp Ser His Tyr Arg Gly Gln Gly
        115                 120                 125

Thr Asp Val Thr Val Ser Ser
    130                 135

<210> 17
<211> 363
<212> DNA
<213> Artificial Sequence

<220>
<223> Description of Combined DNA/RNA Molecule: scaffold
      with Vhh 1BZQ CDR regions

<220>
<223> Description of Artificial Sequence: scaffold with
      Vhh 1BZQ CDR regions

<220>
<221> CDS
<222> (1)..(363)

<400> 17
aat gtg aaa ctg gtt gaa aaa ggt ggc aat ttc gtc gaa aac gat gac      48
Asn Val Lys Leu Val Glu Lys Gly Gly Asn Phe Val Glu Asn Asp Asp
  1               5                  10                  15

gat ctt aag ctc acg tgc cgt gct agc ggt tac gcc tac acg tat atc      96
Asp Leu Lys Leu Thr Cys Arg Ala Ser Gly Tyr Ala Tyr Thr Tyr Ile
             20                  25                  30

tac atg ggt tgg ttc cgt cag gcg ccg aac gac gac agt act aac gtg     144
Tyr Met Gly Trp Phe Arg Gln Ala Pro Asn Asp Asp Ser Thr Asn Val
         35                  40                  45

gcc acc atc gac tcg ggt ggc ggc ggt acc ctg tac ggt gac tcc gtc     192
Ala Thr Ile Asp Ser Gly Gly Gly Gly Thr Leu Tyr Gly Asp Ser Val
     50                  55                  60

aaa gag cgc ttc gat atc cgt cgc gac aaa ggc tcc aac acc gtt acc     240
Lys Glu Arg Phe Asp Ile Arg Arg Asp Lys Gly Ser Asn Thr Val Thr
 65                  70                  75                  80

tta tcg atg gac gat ctg caa ccg gaa gac tct gca gaa tac aat tgt     288
Leu Ser Met Asp Asp Leu Gln Pro Glu Asp Ser Ala Glu Tyr Asn Cys
                 85                  90                  95

gca gcg ggt ggc tac gaa ctg cgc gac cgc acc tac ggt cag cgt ggt     336
Ala Ala Gly Gly Tyr Glu Leu Arg Asp Arg Thr Tyr Gly Gln Arg Gly
            100                 105                 110

cag ggt acc gac gtt acc gtc tcg tcg                                 363
Gln Gly Thr Asp Val Thr Val Ser Ser
        115                 120


<210> 18
<211> 121
<212> PRT
<213> Artificial Sequence
<223> Description of Artificial Sequence: scaffold with
      Vhh 1BZQ CDR regions

<400> 18
Asn Val Lys Leu Val Glu Lys Gly Gly Asn Phe Val Glu Asn Asp Asp
  1               5                  10                  15

Asp Leu Lys Leu Thr Cys Arg Ala Ser Gly Tyr Ala Tyr Thr Tyr Ile
             20                  25                  30

Tyr Met Gly Trp Phe Arg Gln Ala Pro Asn Asp Asp Ser Thr Asn Val
        35                  40                  45

Ala Thr Ile Asp Ser Gly Gly Gly Gly Thr Leu Tyr Gly Asp Ser Val
    50                  55                  60

Lys Glu Arg Phe Asp Ile Arg Arg Asp Lys Gly Ser Asn Thr Val Thr
65                  70                  75                  80

Leu Ser Met Asp Asp Leu Gln Pro Glu Asp Ser Ala Glu Tyr Asn Cys
                85                  90                  95

Ala Ala Gly Gly Tyr Glu Leu Arg Asp Arg Thr Tyr Gly Gln Arg Gly
            100                 105                 110

Gln Gly Thr Asp Val Thr Val Ser Ser
        115                 120

<210> 19
<211> 351
<212> DNA
<213> Artificial Sequence

<220>
<223> Description of Combined DNA/RNA Molecule: scaffold
      with Vhh 1HCV CDR regions

<220>
<223> Description of Artificial Sequence: scaffold with
      Vhh 1HCV CDR regions

<220>
<221> CDS
<222> (1)..(351)

<400> 19
aat gtg aaa ctg gtt gaa aaa ggt ggc aat ttc gtc gaa aac gat gac      48
Asn Val Lys Leu Val Glu Lys Gly Gly Asn Phe Val Glu Asn Asp Asp
1               5                   10                  15

gat ctt aag ctc acg tgc cgt gct gaa ggt cgt acg ggt tcg acc tac      96
Asp Leu Lys Leu Thr Cys Arg Ala Glu Gly Arg Thr Gly Ser Thr Tyr
            20                  25                  30

gat atg ggt tgg ttc cgt cag gcg ccg aac gac gac agt act aac gtg      144
Asp Met Gly Trp Phe Arg Gln Ala Pro Asn Asp Asp Ser Thr Asn Val
        35                  40                  45

gcc acg atc aac tgg gat agc gcc cgt acg tac tac ggt gac tcc gtc      192
Ala Thr Ile Asn Trp Asp Ser Ala Arg Thr Tyr Tyr Gly Asp Ser Val
    50                  55                  60

aaa gag cgc ttc gat atc cgt cgc gac aat gcc tcc aac acc gtt acc      240
Lys Glu Arg Phe Asp Ile Arg Arg Asp Asn Ala Ser Asn Thr Val Thr
65                  70                  75                  80

tta tcg atg gac gat ctg caa ccg gaa gac tct gca gaa tac aat tgt      288
Leu Ser Met Asp Asp Leu Gln Pro Glu Asp Ser Ala Glu Tyr Asn Cys
                85                  90                  95

gca ggt ggt gaa ggc ggc acc tgg gat agc cgt ggt cag ggt acc gac      336
Ala Gly Gly Glu Gly Gly Thr Trp Asp Ser Arg Gly Gln Gly Thr Asp
            100                 105                 110

gtt acc gtc tcg tcg                                                  351
Val Thr Val Ser Ser

        115

<210> 20
<211> 117
<212> PRT
<213> Artificial Sequence
<223> Description of Artificial Sequence: scaffold with
      Vhh 1HCV CDR regions

<400> 20
Asn Val Lys Leu Val Glu Lys Gly Gly Asn Phe Val Glu Asn Asp Asp
  1               5                  10                  15

Asp Leu Lys Leu Thr Cys Arg Ala Glu Gly Arg Thr Gly Ser Thr Tyr
             20                  25                  30

Asp Met Gly Trp Phe Arg Gln Ala Pro Asn Asp Asp Ser Thr Asn Val
         35                  40                  45

Ala Thr Ile Asn Trp Asp Ser Ala Arg Thr Tyr Tyr Gly Asp Ser Val
     50                  55                  60

Lys Glu Arg Phe Asp Ile Arg Arg Asp Asn Ala Ser Asn Thr Val Thr
 65                  70                  75                  80

Leu Ser Met Asp Asp Leu Gln Pro Glu Asp Ser Ala Glu Tyr Asn Cys
                 85                  90                  95

Ala Gly Gly Glu Gly Gly Thr Trp Asp Ser Arg Gly Gln Gly Thr Asp
            100                 105                 110

Val Thr Val Ser Ser
        115



<210> 21
<211> 363
<212> DNA
<213> Artificial Sequence

<220>
<223> Description of Combined DNA/RNA Molecule: primary
      scaffold

<220>
<223> Description of Artificial Sequence: primary
      scaffold

<220>
<221> CDS
<222> (1)..(363)
<223> /note="N stands for unknown"

<400> 21
aat gtg aaa ctg gtt gaa aaa ggt ggc aat ttc gtc gaa aac gat gac      48
Asn Val Lys Leu Val Glu Lys Gly Gly Asn Phe Val Glu Asn Asp Asp
  1               5                  10                  15

gat ctt aag ctc acg tgc cgt gct nnn nnn nnn nnn nnn nnn nnn nnn      96
Asp Leu Lys Leu Thr Cys Arg Ala Xaa Xaa Xaa Xaa Xaa Xaa Xaa Xaa
             20                  25                  30

nnn atg ggt tgg ttc cgt cag gcg ccg aac gac gac agt act aac gtg      144
Xaa Met Gly Trp Phe Arg Gln Ala Pro Asn Asp Asp Ser Thr Asn Val
         35                  40                  45

gcc acc atc gac nnn nnn nnn nnn nnn nnn nnn tac ggt gac tcc gtc     192
Ala Thr Ile Asp Xaa Xaa Xaa Xaa Xaa Xaa Xaa Tyr Gly Asp Ser Val
     50                  55                  60

aaa gag cgc ttc gat atc cgt cgc gac aaa ggc tcc aac acc gtt acc     240
Lys Glu Arg Phe Asp Ile Arg Arg Asp Lys Gly Ser Asn Thr Val Thr
 65                  70                  75                  80

tta tcg atg gac gat ctg caa ccg gaa gac tct gca gaa tac aat tgt     288
Leu Ser Met Asp Asp Leu Gln Pro Glu Asp Ser Ala Glu Tyr Asn Cys
                 85                  90                  95

gca nnn nnn nnn nnn nnn nnn nnn nnn nnn nnn nnn nnn nnn nnn ggt     336
Ala Xaa Xaa Xaa Xaa Xaa Xaa Xaa Xaa Xaa Xaa Xaa Xaa Xaa Xaa Gly
            100                 105                 110

cag ggt acc gac gtt acc gtc tcg tcg                                 363
Gln Gly Thr Asp Val Thr Val Ser Ser
        115                 120


<210> 22
<211> 121
<212> PRT
<213> Artificial Sequence
<223> Description of Artificial Sequence: primary
      scaffold

<400> 22
Asn Val Lys Leu Val Glu Lys Gly Gly Asn Phe Val Glu Asn Asp Asp
  1               5                  10                  15

Asp Leu Lys Leu Thr Cys Arg Ala Xaa Xaa Xaa Xaa Xaa Xaa Xaa Xaa
             20                  25                  30

Xaa Met Gly Trp Phe Arg Gln Ala Pro Asn Asp Asp Ser Thr Asn Val
         35                  40                  45

Ala Thr Ile Asp Xaa Xaa Xaa Xaa Xaa Xaa Xaa Tyr Gly Asp Ser Val
     50                  55                  60

Lys Glu Arg Phe Asp Ile Arg Arg Asp Lys Gly Ser Asn Thr Val Thr
 65                  70                  75                  80

Leu Ser Met Asp Asp Leu Gln Pro Glu Asp Ser Ala Glu Tyr Asn Cys
                 85                  90                  95

Ala Xaa Xaa Xaa Xaa Xaa Xaa Xaa Xaa Xaa Xaa Xaa Xaa Xaa Xaa Gly
            100                 105                 110

Gln Gly Thr Asp Val Thr Val Ser Ser
        115                 120